(19) Europäisches Patentamt
European Patent Office
Office européen des brevets

(11) Veröffentlichungsnummer: **0 438 717 A2**

# (12) EUROPÄISCHE PATENTANMELDUNG

(21) Anmeldenummer: **90124274.3**

(22) Anmeldetag: **14.12.90**

(51) Int. Cl.[5]: **C07D 253/075**, A01N 43/707

(30) Priorität: **26.01.90 DE 4002297**

(43) Veröffentlichungstag der Anmeldung: **31.07.91 Patentblatt 91/31**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Uhr, Hermann, Dr.**
**Düsseldorfer Strasse 67**
**W-5090 Leverkusen 3(DE)**
Erfinder: **Widdig, Arno, Dr.**
**Eifgenstrasse 8**
**W-5068 Odenthal(DE)**
Erfinder: **Berg, Dieter, Dr.**
**Gellertweg 27**
**W-5600 Wuppertal 1(DE)**
Erfinder: **Hänssler, Gerd, Dr.**
**Am Arenzberg 58a**
**W-5090 Leverkusen 3(DE)**

## (54) Trisubstituierte 1,2,4-Triazin-3,5-dione und neue Zwischenprodukte.

(57) Neu sind die tri- und disubstituierten 1,2,4-Triazin-3,5-dione und die disubstituierten 3-Thioxo-1,2,4-triazin-5-one der Formeln (I), (IIa) und (IVa)

**EP 0 438 717 A2**

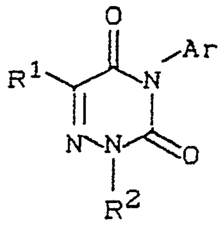

(I) ,

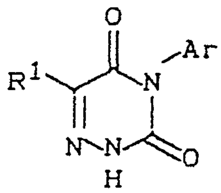

(IIa) ,

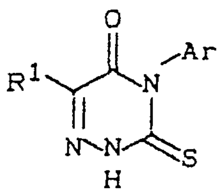

(IVa) ,

die die in der Beschreibung gegebenen Bedeutungen haben, die Verwendung von (I) als Fungizide, von (IIa) und (IVa) als Ausgangsverbindungen zur Herstellung hochaktiver Verbindungen.

Die Verbindungen sind durch die Formeln (I), (IIa) und (IVa) allgemein definiert und können alle nach Analogieverfahren hergestellt werden, z.B. indem man geeignete disubstituierte Verbindungen mit Alkylierungsmiteln umsetzt: die disubstituierten Dione werden aus geeigneten Thioxo-dionen mit geeigneten Entschwefelungsmitteln hergestellt und die Thioxo-dione aus geeigneten $\alpha$-Ketocarbonsäuren mit geeigneten Semicarbaziden.

## TRISUBSTITUIERTE 1,2,4-TRIAZIN-3,5-DIONE UND NEUE ZWISCHENPRODUKTE

Die Erfindung betrifft neue trisubstituierte 1,2,4-Triazin-3,5-dione, Verfahren zu ihrer Herstellung und ihre Verwendung zur Schädlingsbekämpfung, vor allem als Fungizide und neue Zwischenprodukte.

Es ist bereits bekannt geworden, daß 2-Phenyl-1,2,4-triazin-3,5-dione als selektive Herbizide geeignet sind (WO 8600-072-A).

Außerdem sind diverse 1-Aryl-6-azaurazile bekannt, die sich zur Bekämpfung von Coccidiose eignen (z.B. Miller, Max. W. et. al., J. Med. Chem., 24, 1337 (1981); BE 815-246; US 4631-278-A).

Zusätzlich ist noch bekannt geworden, daß 6-Azathymin als Zusatzkomponente bei der Tumorbehandlung mit 5-Fluoruracil geeignet ist (J 55-111 420).

Bislang sind keine 1,2,4-Triazin-3,5-dione bekannt, die sich als Fungizide eignen.

Außerdem ist bereits bekannt geworden, daß strukturell ähnliche trisubstiutierte 1,3,5-Triazin-2,4,6-trione (vgl. DE-OS 3 618 662) eine fungizide Wirkung besitzen.

Die Wirksamkeit dieser Verbindungen ist jedoch insbesondere bei niedrigen Aufwandmengen und Konzentrationen nicht in allen Anwendungsgebieten völlig zufriedenstellend.

Es wurden neue trisubstituierte 1,2,4-Triazin-3,5-dione der allgemeinen Formel (I)

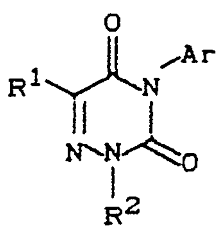

(I)

in welcher

Ar für gegebenenfalls substituiertes Aryl steht,

$R^1$ für einen gegebenenfalls substituierten aliphatischen oder cycloaliphatischen Rest steht und

$R^2$ für einen gegebenenfalls substituierten aliphatischen Rest steht,

gefunden.

Weiterhin wurde gefunden, daß man die neuen trisubstituierten 1,2,4-Triazin-3,5-dione der allgemeinen Formel

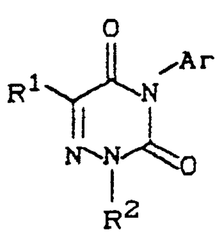

(I)

in welcher

Ar für gegebenenfalls substituiertes Aryl steht,

$R^1$ für einen gegebenenfalls substituierten aliphatischen oder cycloaliphatischen Rest steht und

$R^2$ für einen gegebenenfalls substituierten aliphatischen Rest steht,

erhält,

wenn man die ebenfalls teilweise neuen disubstituierten 1,2,4-Triazin-3,5-dione der allgemeinen Formel (II)

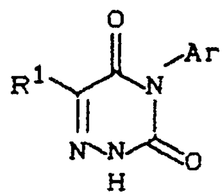

(II)

wobei Ar und $R^1$ die oben angegebene Bedeutung haben, mit Verbindungen der allgemeinen Formel (III)

$R^2$ - X (III)

in welcher

$R^2$ die oben angegebene Bedeutung hat und

X eine Abgangsgruppe, wie z.B. Halogen, Sulfat, Mesylat oder Tosylat,

bedeutet,

gegebenenfalls in Gegenwart eines Verdünnungsmittels und gegebenenfalls in Gegenwart eines Säurebindemittels umsetzt.

Schließlich wurde gefunden, daß die neuen trisubstituierten 1,2,4-Triazin-3,5-dione der Formel (I) sehr gute biologische Eigenschaften besitzen und vor allem zur selektiven Bekämpfung von Schadpilzen insbesondere im Reis geeignet sind.

Die erfindungsgemäßen trisubstituierten 1,2,4-Triazin-3,5-dione sind durch die Formel (I) allgemein definiert. Bevorzugt sind Verbindungen der Formel (I), in welcher

Ar für Phenyl steht, welches gegebenenfalls ein- bis fünffach, gleich oder verschieden substituiert ist durch Halogen, Alkyl mit 1 bis 10 Kohlenstoffatomen, Alkenyl mit 2 bis 10 Kohlenstoffatomen, Alkinyl mit 2 bis 10 Kohlenstoffatomen, Halogenalkyl mit 1 bis 8 Kohlenstoffatomen und 1 bis 8 gleichen oder verschiedenen Halogenatomen, Alkoxy mit 1 bis 10 Kohlenstoffatomen, Halogenalkoxy mit 1 bis 8 Kohlenstoffatomen und 1 bis 8 gleichen oder verschiedenen Halagenatomen, Alkylthio mit 1 bis 10 Kohlenstoffatomen, Halogenalkylthio mit 1 bis 8 Kohlenstoffatomen und 1 bis 8 gleichen oder verschiedenen Halogenatomen, Amino, Monoalkylamino mit geradkettigen oder verzweigten Alkylresten mit 1 bis 6 Kohlenstoffatomen, Dialkylamino mit gleichen oder verschiedenen, geradkettigen oder verzweigten Alkylresten mit je 1 bis 6 Kohlenstoffatomen, Cycloalkyl oder Cycloalkenyl mit 3 bis 8 Kohlenstoffatomen, welche gegebenenfalls ein- bis sechsfach, gleich oder verschieden substituiert sind durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen oder Halogenalkoxy mit 1 bis 4 Kohlenstoffatomen und jeweils 1 bis 9 gleichen oder verschiedenen Halogenatomen, Alkylthio mit 1 bis 4 Kohlenstoffatomen und Halogenalkylthio mit 1 bis 4 Kohlenstoffatomen und 1 bis 5 gleichen oder verschiedenen Halogenatomen,

$R^1$ für geradkettiges oder verzweigtes Alkyl mit 1 bis 12 Kohlenstoffatomen, geradkettiges oder verzweigtes Alkenyl mit 3 bis 12 Kohlenstoffatomen oder geradkettiges oder verzweigtes Alkinyl mit 3 bis 12 Kohlenstoffatomen steht, welche gegebenenfalls ein- bis mehrfach, gleich oder verschieden substituiert sind durch Halogen, Alkoxy mit 1 bis 4 Kohlenstoffatomen, Halogenalkoxy mit 1 bis 4 Kohlenstoffatomen und 1 bis 9 gleichen oder verschiedenen Halogenatomen, Alkylthio mit 1 bis 4 Kohlenstoffatomen, Halogenalkylthio mit 1 bis 4 Kohlenstoffatomen und 1 bis 9 gleichen oder verschiedenen Halogenatomen; weiterhin

$R^1$ für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 4 Kohlenstoffatomen, Halogenalkyl oder Halogenalkoxy mit 1 bis 4 Kohlenstoffatomen und 1 bis 9 gleichen oder verschiedenen Halogenatomen, Alkylthio mit 1 bis 4 Kohlenstoffatomen und Halogenalkylthio mit 1 bis 4 Kohlenstoffatomen und 1 bis 9 gleichen oder verschiedenen Halogenatomen,

$R^2$ für Alkyl mit 1 bis 10 Kohlenstoffatomen, Alkenyl mit 3 bis 10 Kohlenstoffatomen, Alkinyl mit 3 bis 10 Kohlenstoffatomen, Alkoxyalkyl mit je 1 bis 4 Kohlenstoffatomen im Alkoxy und im Alkylteil, Alkylthioalkyl mit je 1 bis 4 Kohlenstoffatomen im Alkylthioteil und im Alkylteil, Alkoxycarbonylalkyl mit 1 bis 4 Kohlenstoffatomen im Alkoxyteil und 1 bis 3 Kohlenstoffatomen im Alkylteil oder für Cyanoalkyl mit 1 bis 5 Kohlenstoffatomen im Alkylteil steht.

Besonders hervorzuheben sind Verbindungen der Formel (I), in welcher

Ar für Phenyl steht, welches gegebenenfalls ein- bis vierfach, gleich oder verschieden substituiert sein kann durch Fluor, Chlor, Brom, Alkyl mit 1 bis 9 Kohlenstoffatomen, Alkenyl mit 2 bis 9 Kohlenstoffatomen, Alkinyl mit 2 bis 9 Kohlenstoffatomen, Halogenalkyl mit 1 bis 7 Kohlenstoffatomen und 1 bis 7 Chlor- und/oder Fluoratomen, Alkoxy mit 1 bis 9 Kohlenstoffatomen, Halogenalkoxy mit 1 bis 7 Kohlenstoffatomen und 1 bis 7 Chlor- und/oder Fluoratomen, Alkylthio mit 1 bis 9 Kohlenstoffatomen, Halogenalkylthio mit 1 bis 7 Kohlenstoffatomen und 1 bis 7 Chlor- und/oder Fluoratomen, Amino, Monoalkylamino mit geradkettigem oder verzweigtem Alkylrest mit 1 bis 5 Kohlenstoffatomen, Dialkylamino mit gleichen oder verschiedenen, geradkettigen oder verzweigten Alkylresten mit je 1 bis 5 Kohlenstoffatomen, Cycloalkyl oder Cycloalkenyl mit 3 bis 7 Kohlenstoffatomen, welches gegebenenfalls ein- bis fünffach, gleich oder verschieden substituiert ist durch Chlor und/oder Fluor, Alkyl mit 1 bis 3 Kohlenstoffatomen, Alkoxy mit 1 bis 3 Kohlenstoffatomen, Halogenalkoxy mit 1 bis 3 Kohlenstoffatomen und 1 bis 5 Chlor- und/oder Fluoratomen, Alkylthio mit 1 bis 3 Kohlenstoffatomen und Halogenalkylthio mit 1 bis 3 Kohlenstoffatomen und 1 bis 5 Chlor- und/oder Fluoratomen,

$R^1$ für geradkettiges oder verzweigtes Alkyl mit 1 bis 10 Kohlenstoffatomen, geradkettiges oder verzweigtes Alkenyl mit 3 bis 10 Kohlenstoffatomen oder geradkettiges oder verzweigtes Alkinyl mit 3 bis 10 Kohlenstoffatomen steht, welche jeweils gegebenenfalls ein- bis dreifach, gleich oder verschieden substituiert sind durch Chlor und/oder Fluor, Alkoxy mit 1 bis 3 Kohlenstoffatomen, Halogenalkoxy mit 1 bis 3 Kohlenstoffatomen und 1 bis 3 Chlor-und/oder Fluoratomen, Alkylthio mit 1 bis 3 Kohlenstoffatomen, Halogenalkylthio mit 1 bis 3 Kohlenstoffatomen und 1 bis 3 Chlor- und/oder Fluoratomen;

$R^1$ weiterhin für Cycloalkyl mit 3 bis 7 Kohlenstoffatomen steht, welches gegebenenfalls ein- bis sechsfach, gleich oder verschieden substituiert ist durch Fluor, Chlor, Alkyl mit 1 bis 3 Kohlenstoffatomen, Alkoxy mit 1 bis 3 Kohlenstoffatomen, Halogenalkoxy mit 1 bis 3 Kohlenstoffatomen und 1 bis 3 Chlor- und/oder Fluoratomen, Alkylthio mit 1 bis 3 Kohlenstoffatomen und Halogenalkylthio mit 1 bis 3 Kohlenstoffatomen und 1 bis 3 Chlor- und/oder Fluoratomen,

$R^2$ für Alkyl mit 1 bis 9 Kohlenstoffatomen, Alkenyl mit 3 bis 9 Kohlenstoffatomen, Alkinyl mit 3 bis 9 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht.

Ganz besonders hervorzuheben sind Verbindungen der Formel (I), in denen

Ar für Phenyl steht, welches gegebenenfalls ein- bis dreifach, gleich oder verschieden substituiert sein kann durch Fluor, Chlor, Brom, Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 6 Kohlenstoffatomen und 1 bis 5 Chlor- und/oder Fluoratomen, Alkoxy mit 1 bis 8 Kohlenstoffatomen, Halogenalkoxy mit 1 bis 6 Kohlenstoffatomen und 1 bis 5 Chlor- und/oder Fluoratomen, Alkylthio mit 1 bis 8 Kohlenstoffatomen, Halogenalkylthio mit 1 bis 6 Kohlenstoffatomen und 1 bis 5 Chlor- und/oder Fluoratomen, Amino, Monoalkylamino mit geradkettigem oder verzweigtem Alkylrest mit 1 bis 4 Kohlenstoffatomen, Dialkylamino mit gleichen oder verschiedenen, geradkettigen oder verzweigten Alkylresten mit je 1 bis 4 Kohlenstoffatomen, Cycloalkyl oder Cycloalkenyl mit 3 bis 6 Kohlenstoffatomen, welche gegebenenfalls ein- bis vierfach, gleich oder verschieden substituiert sind durch Chlor, Fluor, Alkyl mit 1 bis 3 Kohlenstoffatomen, Alkoxy mit 1 bis 3 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen und Halogenalkylthio mit 1 oder 2 Kohlenstoffatomen und 1 bis 5 Chlor- und/oder Fluoratomen,

$R^1$ für geradkettiges oder verzweigtes Alkyl mit 1 bis 9 Kohlenstoffatomen, geradkettiges oder verzweigtes Alkenyl mit 3 bis 9 Kohlenstoffatomen oder geradkettiges oder verzweigtes Alkinyl mit 3 bis 9 Kohlenstoffatomen steht, welche jeweils gegebenenfalls ein- bis zweifach, gleich oder verschieden substituiert sind durch Chlor, Fluor, Alkoxy mit 1 bis 3 Kohlenstoffatomen, Halogenalkoxy mit 1 bis 3 Kohlenstoffatomen und 1 bis 3 Chlor- und/oder Fluoratomen, Alkylthio mit 1 bis 3 Kohlenstoffatomen, Halogenalkylthio mit 1 bis 3 Kohlenstoffatomen und 1 bis 3 Chlor- und/oder Fluoratomen;

$R^1$ weiterhin für Cycloalkyl mit 3 bis 7 Kohlenstoffatomen steht, welches gegebenenfalls ein- bis fünffach, gleich oder verschieden substituiert ist durch Fluor, Chlor, Alkyl mit 1 oder 2 Kohlenstoffatomen, Alkoxy mit 1 oder 2 Kohlenstoffatomen, Halogenalkoxy mit 1 oder 2 Kohlenstoffatomen und 1 bis 4 Chlor- und/oder Fluoratomen, Alkylthio mit 1 oder 2 Kohlenstoffatomen und Halogenalkylthio mit 1 oder 2 Kohlenstoffatomen und 1 bis 3 Chlor-und/oder Fluoratomen,

$R^2$ für Alkyl mit 1 bis 8 Kohlenstoffatomen, Alkenyl mit 3 bis 8 Kohlenstoffatomen, Alkinyl mit 3 bis 8 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanalkyl mit 1 bis 3 Kohlenstoffatomen im Alkylteil steht.

Setzt man gemäß dem erfindungsgemäßen Verfahren beispielsweise 4-Phenyl-6-tert.-butyl-1,2,4-triazin-3,5-dion und Ethyliodid als Ausgangsstoffe ein, so kann der Reaktionsverlauf durch das folgende Formelschema beschrieben werden.

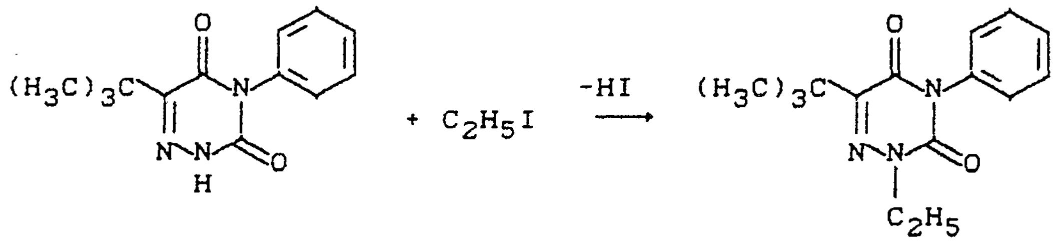


Die Reaktionstemperaturen können bei diesem Verfahren in einem großen Temperaturbereich variiert werden. Im allgemeinen arbeitet man zwischen 20 °C und 150 °C, vorzugsweise zwischen 50 °C und 120 °C.

Bei der Durchführung des Verfahrens setzt man die Ausgangsstoffe und gegebenenfalls das Säurebindemittel in etwa äquimolaren Mengen ein. Ein Überschuß an Säurebindemitteln schadet im allgemeinen nicht.

Die Umsetzungen werden bevorzugt in Gegenwart eines Verdünnungsmittels durchgeführt. Als Verdünnungsmittel kommen alle inerten organischen Lösungsmittel in Frage.

Hierzu gehören vorzugsweise Kohlenwasserstoffe wie Toluol und Xylol; chlorierte Kohlenwasserstoffe wie Chlorbenzol und Chloroform; Ketone wie Aceton, Ether wie Tetrahydrofuran und Dioxan; Nitrile wie Acetonitril.

Als Säurebinder können alle üblichen säurebindenden Mittel verwendet werden. Hierzu gehören vorzugsweise tertiäre Amine wie Triethylamin und Pyridin; Alkalihydroxide wie Natrium- und Kaliumhydroxid und Alkalicarbonate und -hydrogencarbonate wie Kaliumcarbonat und Natriumhydrogencarbonat.

Die Aufarbeitung und Isolierung der erfindungsgemäßen Verbindungen erfolgt in üblicher Weise. Sie fallen entweder sofort kristallin an oder bleiben als Kristallisat oder Öl nach Verdampfen des Lösungsmittels zurück.

Die bei dem erfindungsgemäßen Verfahren als Ausgangsverbindungen benötigten Alkylierungsmittel der Formel (III) sind bekannt. Bevorzugt steht hierbei X für Chlorid, Bromid, Iodid, Tosylat oder Sulfat. $R^2$ hat vorzugsweise diejenige Bedeutung, die bereits im Zusammenhang mit der Beschreibung der erfindungsgemäßen Stoffe der Formel (I) genannt wurde.

Die weiterhin als Ausgangsverbindungen benötigten 1,2,4-Triazin-3,5-dione sind durch die Formel (II) allgemein definiert.

Neu sind die Verbindungen der Formel (IIa) und ebenfalls Teil der Erfindung

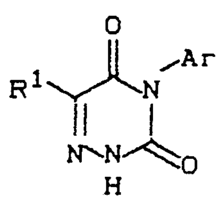

(IIa)

in welcher

Ar für gegebenenfalls substituiertes Aryl steht und

$R^1$ für einen gegebenenfalls substituierten aliphatischen oder cycloaliphatischen Rest steht,

ausgenommen die Verbindungen, in denen Ar für Phenyl oder 4-Methylphenyl und gleichzeitig $R^1$ für

Methyl stehen.

Die neuen und bekannten Verbindungen der Formel (II)

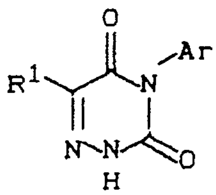

(II)

in welcher

Ar für gegebenenfalls substituiertes Aryl steht und

$R^1$ für einen gegebenenfalls substituierten aliphatischen oder cycloaliphatischen Rest steht,

erhält man, wenn man 3-Thioxo-1,2,4-triazin-5-one der allgemeinen Formel (IV),

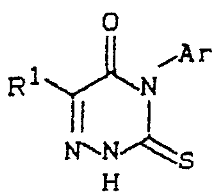

(IV)

in welcher Ar und $R^1$ die oben angegebene Bedeutung haben, mit Entschwefelungsreagenzien, gegebenenfalls in Gegenwart eines Verdünnungsmittels umsetzt.

Die bevorzugten, besonders bevorzugten und ganz besonders bevorzugten Definitionen der Substituenten $R^1$ und Ar in den Formeln (II) bzw. (IIa) entsprechen denen die bei der Formel (I) gegeben wurden.

Setzt man beispielsweise 4-Phenyl-6-tert.-butyl-3-thioxo-1,2,4-triazin-5-on mit $H_2O_2$/KOH als Entschwefelungsreagenz um, so kann der Reaktionsverlauf zur Herstellung der Verbindungen der Formel (II) durch das folgende Reaktionsschema beschrieben werden.

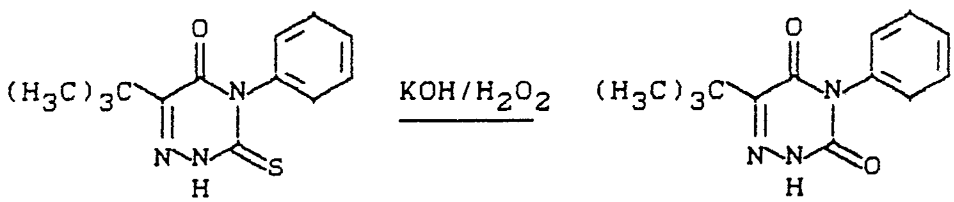


Die Aufarbeitung und Isolierung der Substanzen der Formel (II) erfolgt in üblicher Weise. Sie fallen entweder kristallin an oder lassen sich durch Säuren aus den gegebenenfalls alkalischen Reaktionslösungen ausfällen.

Die verwendeten Entschwefelungsreagenzien sind bekannt (vgl. H. Neunhoeffer, P.F. Wiley; The Chemistry of Heterocyclic compounds; Chemistry of 1,2,3-Triazines and 1,2,4-Triazines, Tetrazines and Pentazines; Wiley-Inter-science, N.Y. 1978, S. 266 und M. Tisler et. al., J. Org. Chem. 25, 770 (1960)). Danach benutzt man u.a. $H_2O_2$ in Gegenwart von KOH oder NaOH; NaOBr, $KMnO_4$, $I_2$/NaOH oder Chloressigsäure.

Die zur Herstellung von Verbindungen der Formel (II) zu verwendenden 3-Thioxo-1,2,4-triazin-5-one sind durch die Formel (IV) allgemein definiert. Neu und Teil der Erfindung sind die Verbindungen der Formel (IVa)

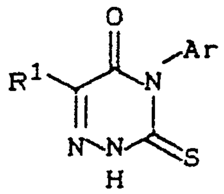

(IVa)

in welcher

Ar für gegebenenfalls substituiertes Aryl steht und

$R^1$ für einen gegebenenfalls substituierten aliphatischen oder cycloaliphatischen Rest steht, ausgenommen die Verbindungen, in denen Ar für Phenyl, 4-Methyl-Phenyl, 3-Methylphenyl, 2-Methoxyphenyl, 3-Methoxyphenyl, 2-Ethoxyphenyl oder 4-Chlorphenyl und gleichzeitig $R^1$ für Methyl stehen.

Die neuen und bekannten 3-Thioxo-1,2,4-triazin-5-one der Formel (IV), in welcher Ar und $R^1$ die angegebene Bedeutung haben, werden hergestellt, indem man α-Ketocarbonsäuren der allgemeinen Formel (V)

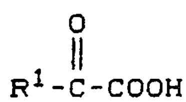

(V)

wobei $R^1$ die oben angegebene Bedeutung hat, mit Thiosemicarbaziden der allgemeinen Formel (VI)

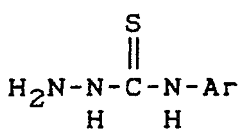

(VI)

wobei Ar die oben angegebene Bedeutung hat,
gegebenenfalls in Gegenwart eines Verdünnungsmittels und gegebenenfalls in Gegenwart einer Säure umsetzt (M.Tisler et.al., J. Org. Chem. 25, 770 (1960)).

Setzt man gemäß diesem Verfahren beispielsweise 4-Phenylthiosemicarbazid und 2-Keto-3,3-dimethylbuttersäure als Ausgangsstoffe ein, so kann der Reaktionsverlauf durch das folgende Formelschema beschrieben werden.

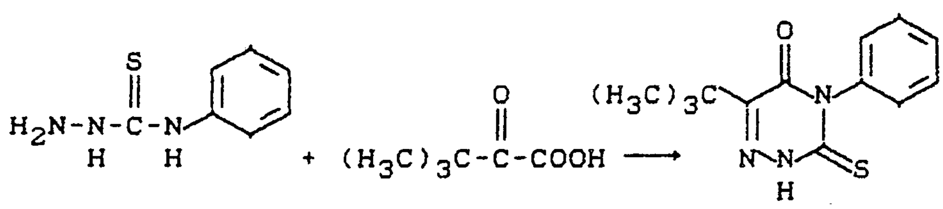

Die benötigten α-Ketocarbonsäuren sind bekannt. $R^1$ hat vorzugsweise diejenige Bedeutung, die bereits im Zusammenhang mit der Beschreibung der Substanzen der allgemeinen Formel (II) und (IV) und der erfindungsgemäßen Wirkstoffe der allgemeinen Formel (I) genannt wurden. Die 4-Aryl-thiosemicarbazide der allgemeinen Formel (VI) sind an sich bekannt und lassen sich in bekannter Weise aus aromatischen Senfölen und Hydrazin erhalten. Ar hat vorzugsweise diejenige Bedeutung, die bereits im Zusammenhang mit der Beschreibung der Substanzen der allgemeinen Formel (II) und (IV) und der erfindungsgemäßen Wirkstoffe der allgemeinen Formel (I) genannt wurden.

Die Reaktionstemperaturen können bei den Verfahren in einem großen Temperaturbereich variiert werden. Im allgemeinen arbeitet man zwischen 20 °C und 150 °C, vorzugsweise zwischen 40 °C und 120 °C. Bei der Durchführung setzt man die Ausgangsstoffe in äquimolaren Mengen ein. Die ä-Ketocarbonsäuren können auch durch Säurezugabe aus ihren entsprechenden Alkalimetallsalzen generiert werden.

Die Umsetzungen werden bevorzugt in Gegenwart eines Verdünnungsmittels durchgeführt. Als Verdünnungsmittel kommen organische Lösungsmittel, Wasser oder Mischungen hieraus in Frage. Vorzugsweise verwendet man Kohlenwasserstoffe wie Toluol oder Xylol, chlorierte Kohlenwasserstoffe wie Chlorbenzol oder Chloroform, Ketone wie Aceton, Ether wie Tetrahydrofuran und Dioxan, Nitrile wie Acetonitril, Alkohole wie Ethanol oder Methanol.

Als Säuren können alle üblichen anorganischen und organischen Säuren verwendet werden. Hierzu gehören bevorzugt Salzsäure, Schwefelsäure, Phosphorsäure, Ameisensäure, Essigsäure und Oxalsäure.

Die Aufarbeitung und Isolierung der Zwischenprodukte der Formel (IV) erfolgt in üblicher Weise. Sie fallen meist kristallin an oder können durch Extraktion aus den Reaktionsmedien gewonnen werden.

Die erfindungsgemäßen Wirkstoffe der Formel (I) weisen eine starke biologische Wirkung auf und können zur Bekämpfung von unerwünschten Schädlingen praktisch eingesetzt werden. Die Wirkstoffe sind für den Gebrauch z.B. als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen,

Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Materialwie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektiziden, Akarizide und in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ulra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen zeigen auch eine Wirkung gegen echten Mehltau an Gurken und Reben und bei entsprechenden Konzentrationen zeigen sie eine blattinsektizide Wirkung.

Herstellungsbeispiele (Beispiel 2)

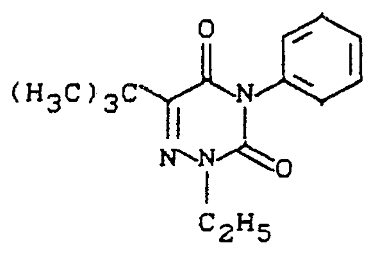

8 g (0,033 Mol) 4-Phenyl-6-tert.-butyl-1,2,4-triazin-3,5-dion, 6,2 g (0,040 Mol) Ethyliodid und 9 g

Kaliumcarbonat werden in 100 ml absolutem Acetonitril 12 Stunden unter Rückfluß gekocht. Nach dem Erkalten filtriert man ab, dampft das Filtrat ein und verreibt mit heißem Wasser. Das kristalline Reaktionsprodukt wird abgesaugt und aus Methanol umkristallisiert. Man erhält 8,65 g (96 % der Theorie) der gewünschten Verbindung. Physikalische Daten siehe Beispiel 2 in der folgenden Tabelle.

Analog dem angegebenen Beispiel oder den im Text beschriebenen Verfahren werden die in der folgenden Tabelle genannten Verbindungen der Formel (I) hergestellt:

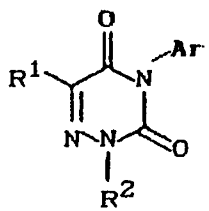

(I)

| Beispiel Nr. | Ar | $R^1$ | $R^2$ | Physikal. Konstanten |
|---|---|---|---|---|
| 1 | 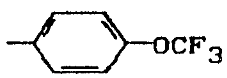 | $-C(CH_3)_3$ | $-C_2H_5$ | Fp.= 96° C |
| 2 | 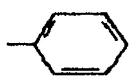 | $-C(CH_3)_3$ | $-C_2H_5$ | Fp.= 80° C |
| 3 | 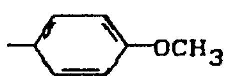 | $-C(CH_3)_3$ | $-CH_3$ | IR: 1721, 1665 br*$cm^{-1}$ |
| 4 | 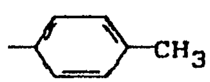 | $-C(CH_3)_3$ | $-C_2H_5$ | Fp.= 120° C |

| Beispiel Nr. | Ar | $R^1$ | $R^2$ | Physikal. Konstanten |
|---|---|---|---|---|
| 5 | 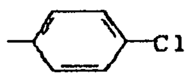 | $-C(CH_3)_3$ | $-C_2H_5$ | Fp.= 146° C |
| 6 | 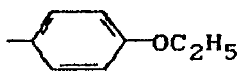 | $-C(CH_3)_3$ | $-CH_3$ | IR: 1719, 1670 br*cm$^{-1}$ |
| 7 | 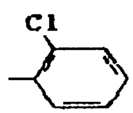 | $-C(CH_3)_3$ | $-CH_3$ | Fp.= 124° C |
| 8 | 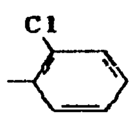 | $-C(CH_3)_3$ | $-C_2H_5$ | Fp.= 92° C |
| 9 | 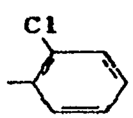 | $-C(CH_3)_3$ | $-C_3H_7$ | Fp.= 58° C |

| Beispiel Nr. | Ar | $R^1$ | $R^2$ | Physikal. Konstanten |
|---|---|---|---|---|
| 10 | 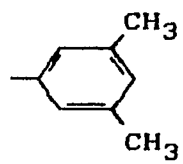 | $-C(CH_3)_3$ | $-CH_3$ | IR: 1719, 1668 $cm^{-1}$ |
| 11 | 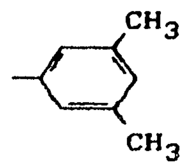 | $-C(CH_3)_3$ | $-C_2H_5$ | Fp.= 126° C |
| 12 | 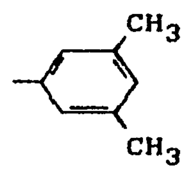 | $-C(CH_3)_3$ | $-CH_2-CH=CH_2$ | Fp.= 140° C |
| 13 | 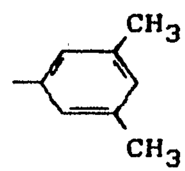 | $-C(CH_3)_3$ | $-CH_2-C\equiv CH$ | Fp.= 156° C |

| Beispiel Nr. | Ar | $R^1$ | $R^2$ | Physikal. Konstanten |
|---|---|---|---|---|
| 14 | 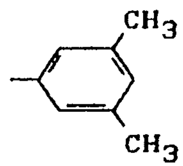 | $-C(CH_3)_3$ | $-CH(CH_3)_2$ | Fp.= 140° C |
| 15 | 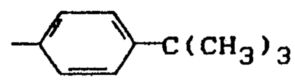 | $-C(CH_3)_3$ | $-CH_3$ | Fp.= 156° C |
| 16 | 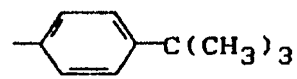 | $-C(CH_3)_3$ | $-C_2H_5$ | IR: 1720, 1671 $cm^{-1}$ |
| 17 | 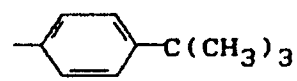 | $-C(CH_3)_3$ | $-CH_2-CH=CH_2$ | IR: 1720, 1650-1670 $cm^{-1}$ |
| 18 | 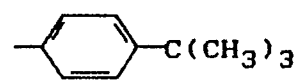 | $-C(CH_3)_3$ | $-C_3H_7$ | IR: 1720, 1670 $cm^{-1}$ |
| 19 | 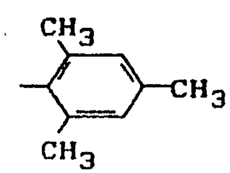 | $-C(CH_3)_3$ | $-CH_3$ | Fp.= 178° C |

| Beispiel Nr. | Ar | $R^1$ | $R^2$ | Physikal. Konstanten |
|---|---|---|---|---|
| 20 | 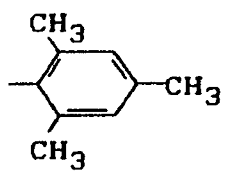 | $-C(CH_3)_3$ | $-C_2H_5$ | Fp.= 86° C |
| 21 | 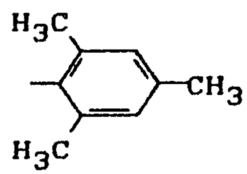 | $-C(CH_3)_3$ | $-C_4H_9$ | Fp.= 95° C |
| 22 | 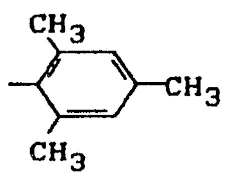 | $-C(CH_3)_3$ | $-C_3H_7$ | Fp.= 90° C |
| 23 | 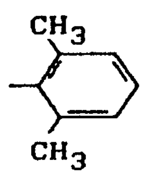 | $-C(CH_3)_3$ | $-C_2H_5$ | Fp.= 118° C |

| Beispiel Nr. | Ar | $R^1$ | $R^2$ | Physikal. Konstanten |
|---|---|---|---|---|
| 24 | 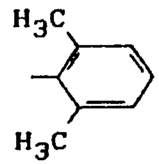 | $-C(CH_3)_3$ | $-C_4H_9$ | IR: 1719, 1668 $cm^{-1}$ |
| 25 | 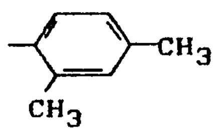 | $-C(CH_3)_3$ | $-C_2H_5$ | Fp.= 100° C |
| 26 | 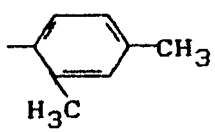 | $-C(CH_3)_3$ | $-C_5H_{11}$ | |
| 27 | 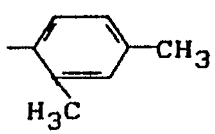 | $-C(CH_3)_3$ | $-CH_2-C{\equiv}CH$ | IR: 1722, 1660-1690 $cm^{-1}$ |
| 28 | 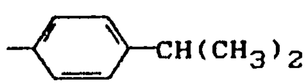 | $-C(CH_3)_3$ | $-CH_3$ | IR: 1718, 1670 $cm^{-1}$ |

| Beispiel Nr. | Ar | $R^1$ | $R^2$ | Physikal. Konstanten |
|---|---|---|---|---|
| 29 | 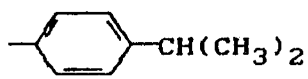 | $-C(CH_3)_3$ | $-C_2H_5$ | IR: 1720, 1670 $cm^{-1}$ |
| 30 | 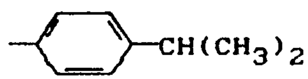 | $-C(CH_3)_3$ | $-CH_2-CH=CH_2$ | IR: 1720, 1675 $cm^{-1}$ |
| 31 | 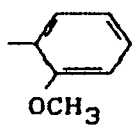 | $-C(CH_3)_3$ | $-CH_3$ | Fp.= 126° C |
| 32 | 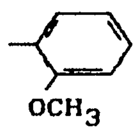 | $-C(CH_3)_3$ | $-C_2H_5$ | Fp.= 100° C |
| 33 | 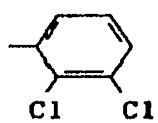 | $-C(CH_3)_3$ | $-C_2H_5$ | Fp.= 92° C |

| Beispiel Nr. | Ar | $R^1$ | $R^2$ | Physikal. Konstanten |
|---|---|---|---|---|
| 34 | 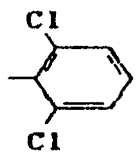 | $-C(CH_3)_3$ | $-CH_2-CH=CH_2$ | Fp.= 90° C |
| 35 | 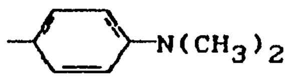 | $-CH(CH_3)_2$ | $-CH_3$ | Fp.= 168° C |
| 36 | 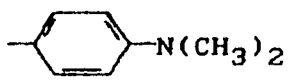 | $-CH(CH_3)_2$ | $-CH_2-CH=CH_2$ | IR: 1721, 1670, 1608 $cm^{-1}$ |
| 37 | 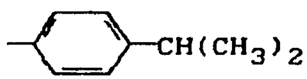 | $-CH(CH_3)_2$ | $-C_2H_5$ | IR: 1720, 1673 $cm^{-1}$ |
| 38 | 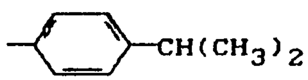 | $-CH(CH_3)_2$ | $-CH_2-CH=CH_2$ | IR: 1721, 1675, 1600 $cm^{-1}$ |
| 39 | 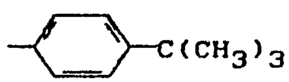 | $-CH(CH_3)_2$ | $-C_2H_5$ | IR: 1721, 1670 br*, 1600 $cm^{-1}$ |

| Beispiel Nr. | Ar | $R^1$ | $R^2$ | Physikal. Konstanten |
|---|---|---|---|---|
| 40 | 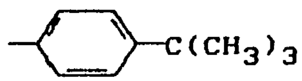 | $-CH(CH_3)_2$ | $-CH_3$ | Fp.= 78° C |
| 41 | 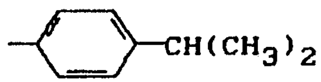 | $-CH(CH_3)_2$ | $-C_3H_7$ | IR: 1720, 1675, 1600 $cm^{-1}$ |
| 42 | 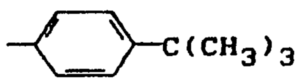 | $-CH(CH_3)_2$ | $-C_3H_7$ | IR: 1720, 1675, 1600 $cm^{-1}$ |
| 43 | 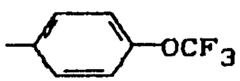 | $-CH(CH_3)_2$ | $-C_2H_5$ | Fp.= 56° C |
| 44 | 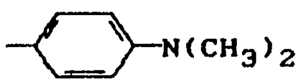 | $-CH(CH_3)_2$ | $-C_2H_5$ | Fp.= 114° C |
| 45 | 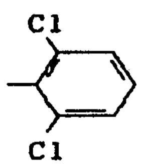 | $-C(CH_3)_3$ | $-C_3H_7$ | Fp.= 88° C |

| Beispiel Nr. | Ar | $R^1$ | $R^2$ | Physikal. Konstanten |
|---|---|---|---|---|
| 46 | 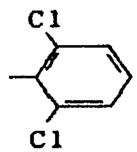 | $-C(CH_3)_3$ | $-CH_3$ | Fp.= 162° C |
| 47 | 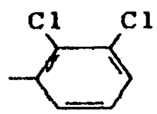 | $-C(CH_3)_3$ | $-CH_3$ | Fp.= 136° C |
| 48 | 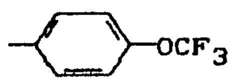 | $-CH(CH_3)_2$ | $-CH_2-CH=CH_2$ | Fp.= 48° C |
| 49 | 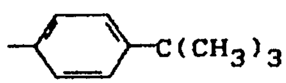 | $-CH(CH_3)_2$ | $-CH_2-CH=CH_2$ | IR: 1720, 1665, 1600 $cm^{-1}$ |
| 50 | 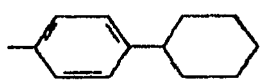 | $-C(CH_3)_3$ | $-CH_2-CH=CH_2$ | IR: 1720, 1670 $cm^{-1}$ |
| 51 | 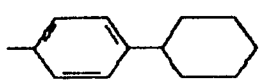 | $-C(CH_3)_3$ | $-C_2H_5$ | IR: 1720, 1670 $cm^{-1}$ |

| Beispiel Nr. | Ar | $R^1$ | $R^2$ | Physikal. Konstanten |
|---|---|---|---|---|
| 52 | 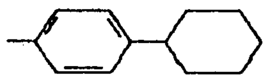 | $-C(CH_3)_3$ | $-CH_3$ | Fp. = 114° C |
| 53 | 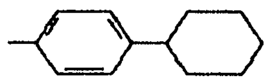 | $-C(CH_3)_3$ | $-C_3H_7$ | Fp. = 118° C |
| 54 | 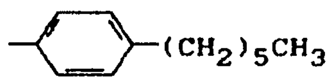 | $-C(CH_3)_3$ | $-CH_3$ | IR: 1720, 1660-1675 $cm^{-1}$ |
| 55 | 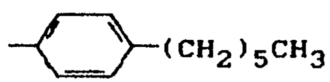 | $-C(CH_3)_3$ | $-C_2H_5$ | Fp. = 40° C |
| 56 | 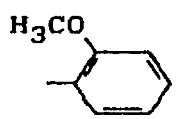 | $-CH(CH_3)_2$ | $-CH_2-\overset{O}{\overset{\|}{C}}-OC_2H_5$ | Fp. = 66° C |
| 57 | 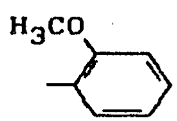 | $-CH(CH_3)_2$ | $-C_2H_5$ | Fp. = 88° C |

| Beispiel Nr. | Ar | $R^1$ | $R^2$ | Physikal. Konstanten |
|---|---|---|---|---|
| 58 | 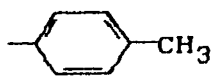 | $-CH(CH_3)_2$ | $-CH_3$ | Fp.= 116° C |
| 59 | 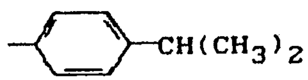 | $-C(CH_3)(C_2H_5)_2$ | $-CH_2-CH=CH_2$ | IR: 1720, 1670 $cm^{-1}$ |
| 60 | 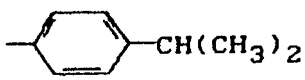 | $-C(CH_3)(C_2H_5)_2$ | $-C_2H_5$ | IR: 1721, 1670-1680 $cm^{-1}$ |
| 61 | 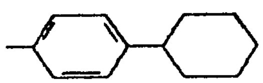 | $-CH(CH_3)_2$ | $-CH_2-CH=CH_2$ | IR: 1720, 1675, 1600 $cm^{-1}$ |
| 62 | 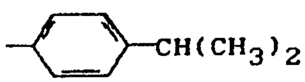 | $-C(CH_3)(C_2H_5)_2$ | $-CH_3$ | IR: 1722, 1670 br*, 1580 $cm^{-1}$ |
| 63 | 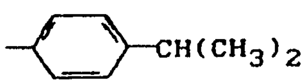 | $-C(CH_3)(C_2H_5)_2$ | $-C_3H_7$ | IR: 1721, 1670 br*, 1580 $cm^{-1}$ |

| Beispiel Nr. | Ar | $R^1$ | $R^2$ | Physikal. Konstanten |
|---|---|---|---|---|
| 64 | 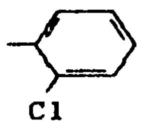 | $-CH(CH_3)_2$ | $-CH_3$ | Fp.= 74° C |
| 65 | 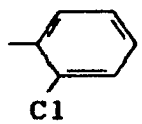 | $-CH(CH_3)_2$ | $-CH(CH_3)_2$ | Fp.= 94° C |
| 66 | 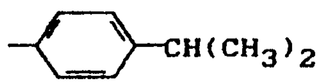 | $-C(CH_3)(C_2H_5)_2$ | $-C_4H_9$ | IR: 1720, 1670, 1580 $cm^{-1}$ |
| 67 | 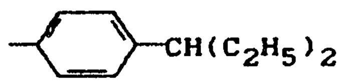 | $-C(CH_3)_3$ | $-C_2H_5$ | IR: 1720, 1670, 1580 $cm^{-1}$ |
| 68 | 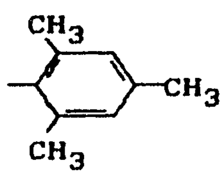 | $-CH_3$ | $-CH_3$ | Fp.= 192° C |

| Beispiel Nr. | Ar | $R^1$ | $R^2$ | Physikal. Konstanten |
|---|---|---|---|---|
| 69 | 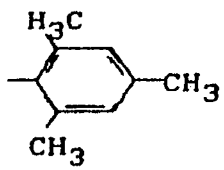 | $-CH_3$ | $-C_4H_9$ | Fp.= 90° C |
| 70 | 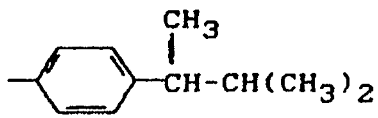 | $-C(CH_3)_3$ | $-C_2H_5$ | IR: 1720, 1678, 1580 $cm^{-1}$ |
| 71 | 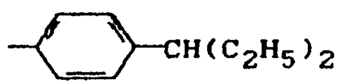 | $-C(CH_3)_3$ | $-CH_2-CH=CH_2$ | IR: 1720, 1670, 1580 $cm^{-1}$ |
| 72 | 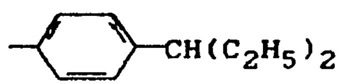 | $-C(CH_3)_3$ | $-CH_3$ | IR: 1720, 1670-1680, br* 1580 $cm^{-1}$ |
| 73 | 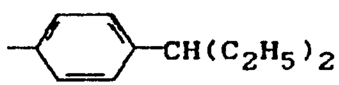 | $-C(CH_3)_3$ | $-C_3H_7$ | IR: 1720, 1675, 1580 $cm^{-1}$ |

| Beispiel Nr. | Ar | $R^1$ | $R^2$ | Physikal. Konstanten |
|---|---|---|---|---|
| 74 | 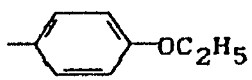 | $-CH(CH_3)_2$ | $-C_4H_9$ | Fp.= 58° C |
| 75 | 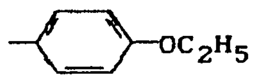 | $-CH(CH_3)_2$ | $-CH_2-CH=CH_2$ | Fp.= 60° |
| 76 | 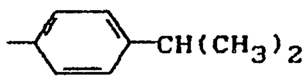 | $-C(CH_3)(C_2H_5)_2$ | $-CH_2-C\equiv CH$ | IR: 1720, 1670 br*, 1585 $cm^{-1}$ |
| 77 | 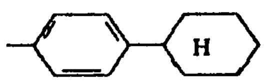 | $-CH(CH_3)_2$ | $-C_2H_5$ | Fp.= 68° C |
| 78 | 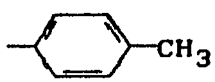 | $-CH(CH_3)_2$ | $-CH_2-C\equiv CH$ | Fp.= 108° C |
| 79 | 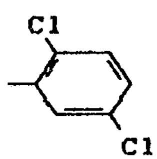 | $-C(CH_3)_3$ | $-CH_3$ | Fp.= 152° C |

| Beispiel Nr. | Ar | $R^1$ | $R^2$ | Physikal. Konstanten |
|---|---|---|---|---|
| 80 | 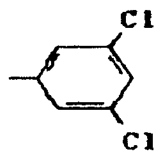 | $-C(CH_3)_3$ | $-CH_3$ | Fp.= 136° C |
| 81 | 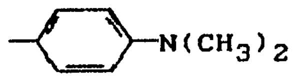 | $-C(CH_3)_3$ | $-C_2H_5$ | Fp.= 96° C |
| 82 | 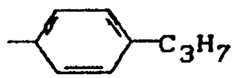 | $-C(CH_3)_3$ | $-CH_3$ | IR: 1720, 1675, 1580 $cm^{-1}$ |
| 83 | 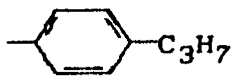 | $-C(CH_3)_3$ | $-C_2H_5$ | Fp.= 74° C |
| 84 | 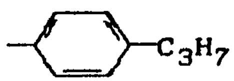 | $-C(CH_3)_3$ | $-C_3H_7$ | Fp.= 70° C |
| 85 | 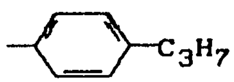 | $-C(CH_3)_3$ | $-C_4H_9$ | IR: 1720, 1672, 1580 $cm^{-1}$ |

| Beispiel Nr. | Ar | $R^1$ | $R^2$ | Physikal. Konstanten |
|---|---|---|---|---|
| 86 | 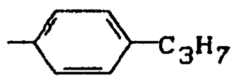 | $-C(CH_3)_3$ | $-CH_2-CH=CH_2$ | Fp. 60° C |
| 87 | 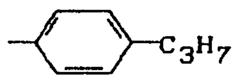 | $-C(CH_3)_3$ | $-CH_2-C\equiv CH_2$ | |
| 88 | 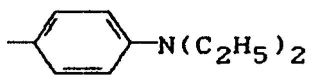 | $-C(CH_3)_3$ | $-CH_3$ | Fp.= 68° C |
| 89 | 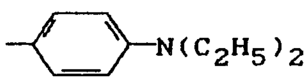 | $-C(CH_3)_3$ | $-C_2H_5$ | Fp. 98° C |
| 90 | 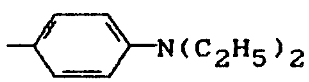 | $-C(CH_3)_3$ | $-C_3H_7$ | IR: 1722, 1675, 1580 $cm^{-1}$ |
| 91 | 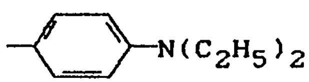 | $-C(CH_3)_3$ | $-CH_2-CH=CH_2$ | Fp.= 92° C |
| 92 | 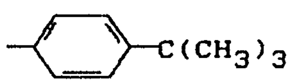 | $-C(CH_3)(C_2H_5)_2$ | $-CH_3$ | IR: 1720, 1672, 1580 $cm^{-1}$ |

| Beispiel Nr. | Ar | $R^1$ | $R^2$ | Physikal. Konstanten |
|---|---|---|---|---|
| 93 | 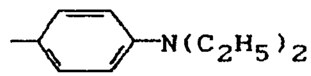 | $-C(CH_3)_3$ | $-C_4H_9$ | Fp=86° C |
| 94 | 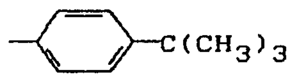 | $-C(C_2H_5)_2CH_3$ | $-C_2H_5$ | IR: 1718, 1670 $cm^{-1}$ |
| 95 | 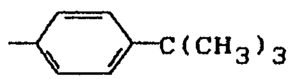 | $-C(C_2H_5)_2CH_3$ | $-C_3H_7$ | IR: 1720, 1670 $cm^{-1}$ |
| 96 | 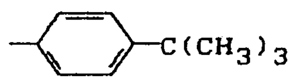 | $-C(C_2H_5)_2CH_3$ | $-CH_2CH=CH_2$ | IR: 1718, 1675, 1410, 1270 $cm^{-1}$ |
| 97 | 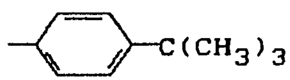 | $-C(C_2H_5)_2CH_3$ | $-C_4H_9$ | IR: 1720, 1675, 1415, 1285 $cm^{-1}$ |
| 98 | 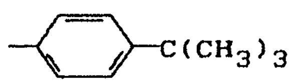 | $-CH(CH_3)(C_2H_5)$ | $-CH_3$ | IR: 1716, 1670 $cm^{-1}$ |

| Beispiel Nr. | Ar | $R^1$ | $R^2$ | Physikal. Konstanten |
|---|---|---|---|---|
| 99 | 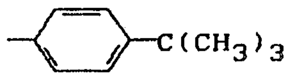 | $-CH(CH_3)(C_2H_5)$ | $-C_4H_9$ | IR: 1720, 1670, 1420, 1290 $cm^{-1}$ |
| 100 | 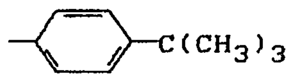 | $-CH_2-CH(CH_3)_2$ | $-C_2H_5$ | IR: 1720, 1670, 1420, 1295 $cm^{-1}$ |
| 101 | 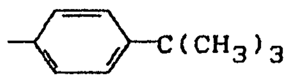 | $-CH_2-CH(CH_3)_2$ | $-C_3H_7$ | IR: 1720, 1670, 1422, 1290 $cm^{-1}$ |
| 102 | 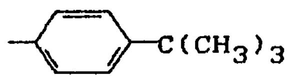 | $-C_3H_7$ | $-C_2H_5$ | IR: 1718, 1660, 1420, 1285 $cm^{-1}$ |
| 103 | 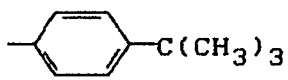 | $-C_3H_7-$ | $-CH(CH_3)_2$ | IR: 1718, 1665, 1420, 1285 $cm^{-1}$ |
| 104 | 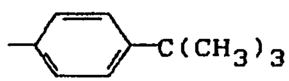 | $-CH(CH_3)(C_2H_5)$ | $-C_2H_5$ | IR: 1710, 1660, 1420, 1290 $cm^{-1}$ |

| Beispiel Nr. | Ar | $R^1$ | $R^2$ | Physikal. Konstanten |
|---|---|---|---|---|
| 105 | 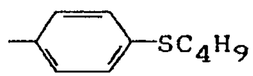 | $-C(CH_3)_3$ | $-CH_3$ | IR: 1720, 1670, 1490, 1430, 1300 $cm^{-1}$ |
| 106 | 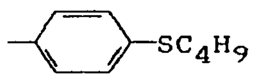 | $-C(CH_3)_3$ | $-C_2H_5$ | IR: 1720, 1665, 1490, 1420, 1280 $cm^{-1}$ |
| 107 | 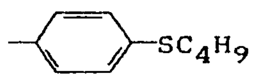 | $-C(CH_3)_3$ | $-C_3H_7$ | IR: 1720, 1670, 1490, 1420, 1280 $cm^{-1}$ |
| 108 | 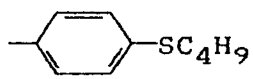 | $-C(CH_3)_3$ | $-CH_2CH=CH_2$ | IR: 1720, 1670, 1495, 1420, 1280 $cm^{-1}$ |
| 109 | 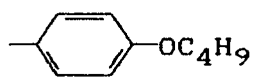 | $-C(CH_3)_3$ | $-C_3H_7$ | IR: 1720, 1670, 1420, 1250 $cm^{-1}$ |
| 110 | 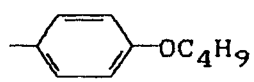 | $-C(CH_3)_3$ | $-C_2H_5$ | IR: 1720, 1660, 1240 $cm^{-1}$ |

| Beispiel Nr. | Ar | $R^1$ | $R^2$ | Physikal. Konstanten |
|---|---|---|---|---|
| 111 | 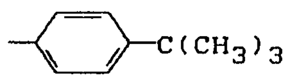 | $-CH_2-CH(CH_3)_2$ | $-CH_3$ | Fp=106° C |
| 112 | 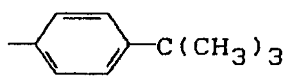 | $-C_3H_7$ | $-CH_2-CH(CH_3)_2$ | IR: 1720, 1670, 1430, 1285 $cm^{-1}$ |
| 113 | 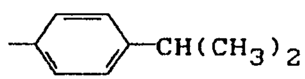 | $-C(CH_3)_2CH_2F$ | $-CH_2CH=CH_2$ | IR: 1720, 1675, 1420, 1280 $cm^{-1}$ |
| 114 | 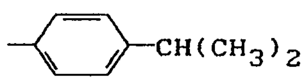 | $-C(CH_3)_2CH(CH_3)_2$ | $-CH_3$ | IR: 1720, 1670, 1430, 1300 $cm^{-1}$ |
| 115 | 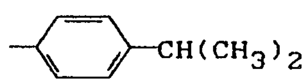 | $-C(CH_3)_2CH(CH_3)_2$ | $-C_2H_5$ | IR: 1720, 1660, 1280 $cm^{-1}$ |
| 116 | 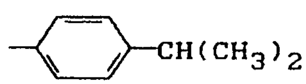 | $-C(CH_3)_2CH(CH_3)_2$ | $-C_3H_7$ | IR: 1720, 1670, 1420, 1280 $cm^{-1}$ |

| Beispiel Nr. | Ar | $R^1$ | $R^2$ | Physikal. Konstanten |
|---|---|---|---|---|
| 117 | 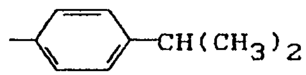 | $-C(CH_3)_2CH(CH_3)_2$ | $-CH_2CH=CH_2$ | IR: 1720, 1670, 1410, 1270 $cm^{-1}$ |
| 118 | 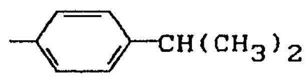 | $-C(CH_3)(C_2H_5)(C_4H_9)$ | $-C_2H_5$ | IR: 1720, 1670, 1420, 1285 $cm^{-1}$ |
| 119 | 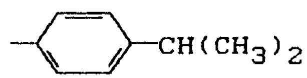 | $-C(CH_3)(C_2H_5)(C_4H_9)$ | $-CH_2CH=CH_2$ | |
| 120 | 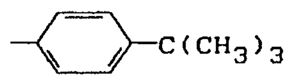 | $-C(CH_3)_2CH(CH_3)_2$ | $-C_2H_5$ | IR: 1720, 1670, 1420, 1280, 760 $cm^{-1}$ |
| 121 | 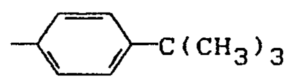 | $-C(CH_3)(C_2H_5)(C_4H_9)$ | $-C_2H_5$ | IR: 1720, 1665, 1415, 1280 $cm^{-1}$ |
| 122 | 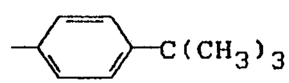 | $-C(CH_3)(C_2H_5)(C_4H_9)$ | $-CH_2CH=CH_2$ | IR: 1720, 1670, 1410, 1270 $cm^{-1}$ |

| Beispiel Nr. | Ar | $R^1$ | $R^2$ | Physikal. Konstanten |
|---|---|---|---|---|
| 123 | 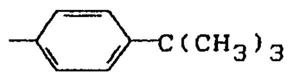 | $-C(CH_3)_2CH(CH_3)_2$ | $-CH_2CH{=}CH_3$ | IR: 1720, 1660, 1410, 1270, 750 $cm^{-1}$ |
| 124 | 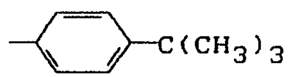 | $-C(CH_3)_2CH(CH_3)_2$ | $-CH_2C{\equiv}CH$ | IR: 1720, 1670, 1420, 1275 $cm^{-1}$ |
| 125 | 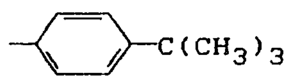 | $-C(CH_3)_2CH_2F$ | $-C_2H_5$ | IR: 1720, 1670, 1420, 1285, 1050 $cm^{-1}$ ($CHCl_3$-Lösung) |
| 126 | 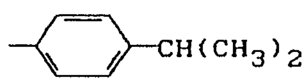 | $-C(CH_3)_2CH_2-O-C_2H_5$ | $-C_2H_5$ | Fp=80° C |
| 127 | 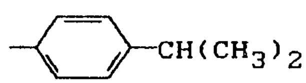 | $-C(CH_3)_2CH_2-O-C_2H_5$ | $-CH_2CH{=}CH_2$ | IR: 1720, 1670, 1410, 1105 $cm^{-1}$ |
| 128 | 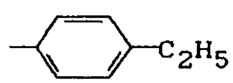 | $-C(CH_3)_3$ | $-C_2H_5$ | Fp=90° C |

| Beispiel Nr. | Ar | $R^1$ | $R^2$ | Physikal. Konstanten |
|---|---|---|---|---|
| 129 | 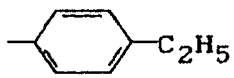 | $-C(CH_3)_3$ | $-C_3H_7$ | Fp.=90° C |
| 130 | 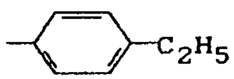 | $-C(CH_3)_3$ | $-CH_2CH=CH_2$ | Fp.=82° C |
| 131 | 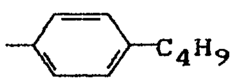 | $-C(CH_3)_3$ | $-C_2H_5$ | Fp.=58° C |
| 132 | 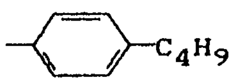 | $-C(CH_3)_3$ | $-C_3H_7$ | Fp.=60° C |
| 133 | 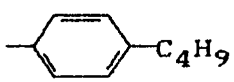 | $-C(CH_3)_3$ | $-CH_2CH=CH_2$ | Fp.=74° C |
| 134 | 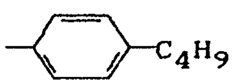 | $-C(CH_3)_3$ | $-CH_2-CH(CH_3)_2$ | Fp.=60° C |

| Beispiel Nr. | Ar | $R^1$ | $R^2$ | Physikal. Konstanten |
|---|---|---|---|---|
| 135 | 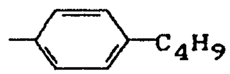 | $-C(CH_3)_3$ | $-CH_2-CH=CHCH_3$ | IR: 1720, 1665, 1420, 1275 $cm^{-1}$ |
| 136 | 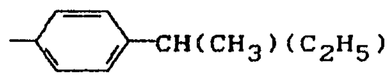 | $-C(CH_3)_3$ | $-CH_3$ | IR: 1720, 1675, 1425, 1300 $cm^{-1}$ |
| 137 | 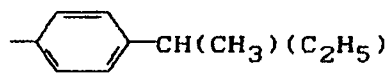 | $-C(CH_3)_3$ | $-C_3H_7$ | IR: 1710, 1660, 1410, 1280 $cm^{-1}$ |
| 138 | 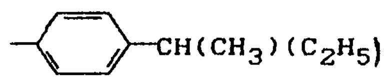 | $-C(CH_3)_3$ | $-CH_2CH=CH_2$ | IR: 1720, 1675, 1420, 1280 $cm^{-1}$ |
| 139 | 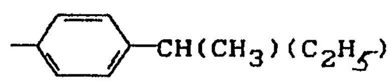 | $-C(CH_3)_3$ | $-C_2H_5$ | IR: 1720, 1670, 1420, 1280 $cm^{-1}$ |
| 140 | 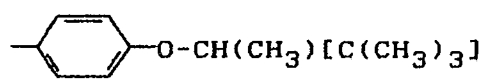 | $-C(CH_3)_3$ | $-CH_3$ | |

| Beispiel Nr. | Ar | $R^1$ | $R^2$ | Physikal. Konstanten |
|---|---|---|---|---|
| 141 | 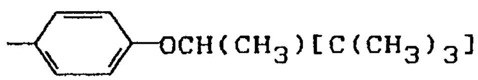 | $-C(CH_3)_3$ | $-C_3H_7$ | IR: 1720, 1670, 1250 $cm^{-1}$ |
| 142 | 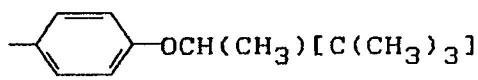 | $-C(CH_3)_3$ | $-C_2H_5$ | IR: 1720, 1660-1680 1250 $cm^{-1}$ |
| 143 | 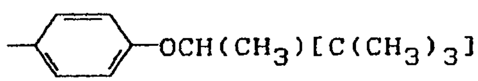 | $-C(CH_3)_3$ | $-CH_2CH=CH_2$ | Fp=104° C |
| 144 | 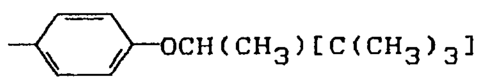 | $-C(CH_3)_3$ | $-CH_2C\equiv CH$ | |
| 145 | 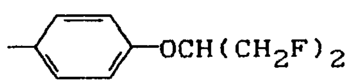 | $-C(CH_3)_3$ | $-C_2H_5$ | Fp=102° C |
| 146 | 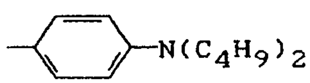 | $-C(CH_3)_3$ | $-CH_3$ | IR: 1718, 1670, 1295, 750 $cm^{-1}$ |

| Beispiel Nr. | Ar | $R^1$ | $R^2$ | Physikal. Konstanten |
|---|---|---|---|---|
| 147 | 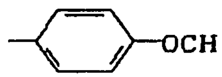 $-OCH(CH_2F)_2$ | $-C(CH_3)_3$ | $-CH_2CH=CH_2$ | IR: 1715, 1660, 1240 $cm^{-1}$ |
| 148 | 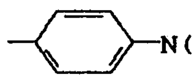 $-N(C_4H_9)_2$ | $-C(CH_3)_3$ | $-C_2H_5$ | IR: 1710, 1660, 1280, 750 $cm^{-1}$ |
| 149 | 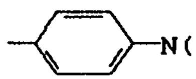 $-N(C_3H_7)_2$ | $-C(CH_3)_3$ | $-CH_2CH=CH_2$ | IR: 1715, 1670, 1520 1400, 1120 $cm^{-1}$ |
| 150 | 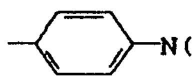 $-N(C_3H_7)_2$ | $-C(CH_3)_3$ | $-C_2H_5$ | IR: 1710, 1665, 1510 $cm^{-1}$ |

br* bedeutet "broad", breite Bande

Herstellungsbeispiele der Formel (II)

(Beispiel Nr. II-2)

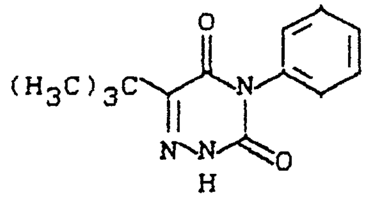

10 g (0,038 Mol) 4-Phenyl-6-tert.-butyl-3-thioxo-1,2,4-triazin-5-on werden in einer Lösung von 6,43 g (0,115 Mol) Kaliumhydroxid in 170 ml Methanol/Wasser = 2:1 gelöst und mit einer Lösung von 12 ml Wasserstoffperoxid in 50 ml Wasser versetzt. Man rührt 35 Minuten bei Raumtemperatur nach und säuert dann mit einer Lösung aus 11 ml konz. Salzsäure in 50 ml Wasser an. Das ausgefallene Reaktionsprodukt wird abgesaugt, mit Wasser gewaschen und getrocknet. Man erhält 9,0 g (85 % der Theorie) an gewünschter Substanz, Physikalische Daten siehe Beispiel Nr. II-2 in der folgenden Tabelle.

Analog dem angegebenen Beispiel oder den im Text beschriebenen Verfahren werden die in der folgenden Tabelle genannten Verbindungen der Formel (II) hergestellt.

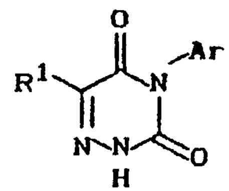

(II)

| Beispiel Nr. | Ar | $R^1$ | Physikal. Konstanten |
|---|---|---|---|
| II-1 | 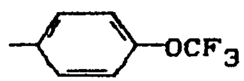 | $-C(CH_3)_3$ | Fp.= 128° C |
| II-2 | 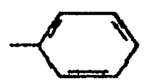 | $-C(CH_3)_3$ | Fp.= 192° C |
| II-3 | 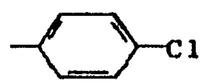 | $-C(CH_3)_3$ | IR: 1722, 1675 $cm^{-1}$ |
| II-4 | 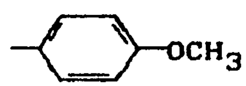 | $-C(CH_3)_3$ | Fp.= 194° C |

| Beispiel Nr. | Ar | $R^1$ | Physikal. Konstanten |
|---|---|---|---|
| II-5 | 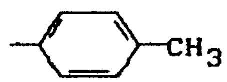 | $-C(CH_3)_3$ | IR: 1720, 1670 $cm^{-1}$ |
| II-6 | 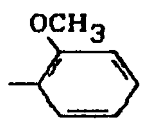 | $-C(CH_3)_3$ | Fp.= 210° C |
| II-7 | 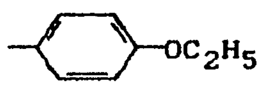 | $-C(CH_3)_3$ | IR: 1720, 1670 $cm^{-1}$ |
| II-8 | 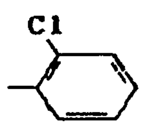 | $-C(CH_3)_3$ | Fp.= 214° C |
| II-9 | 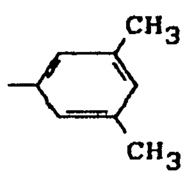 | $-C(CH_3)_3$ | Fp.= 186° C |

| Beispiel Nr. | Ar | $R^1$ | Physikal. Konstanten |
|---|---|---|---|
| II-10 | 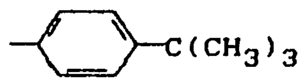 | $-C(CH_3)_3$ | Fp.= 198° C |
| II-11 | 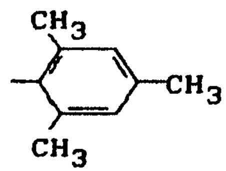 | $-C(CH_3)_3$ | Fp.= 260° C |
| II-12 | 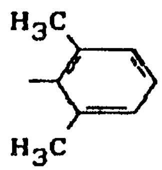 | $-C(CH_3)_3$ | Fp.= 260° C |
| II-13 | 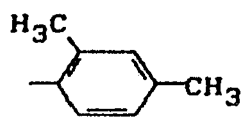 | $-C(CH_3)_3$ | Fp.= 252° C |
| II-14 | 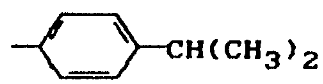 | $-C(CH_3)_3$ | Fp.= 182° C |

| Beispiel Nr. | Ar | $R^1$ | Physikal. Konstanten |
|---|---|---|---|
| II-15 | 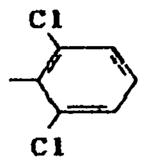 | $-C(CH_3)_3$ | Fp.= 260° C |
| II-16 | 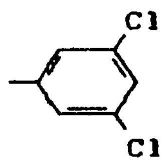 | $-C(CH_3)_3$ | Fp.= 251° C |
| II-17 | 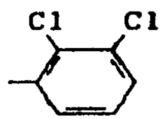 | $-C(CH_3)_3$ | Fp.= 188° C |
| II-18 | 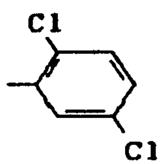 | $-C(CH_3)_3$ | Fp.= 190° C |
| II-19 | 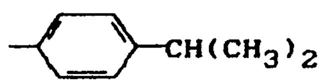 | $-CH(CH_3)_2$ | Fp.= 160° C |

| Beispiel Nr. | Ar | $R^1$ | Physikal. Konstanten |
|---|---|---|---|
| II-20 | 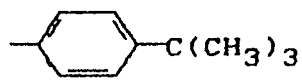 | $-CH(CH_3)_2$ | Fp.= 170° C |
| II-21 | 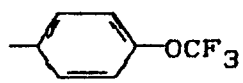 | $-CH(CH_3)_2$ | Fp.= 150° C |
| II-22 | 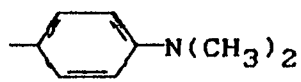 | $-CH(CH_3)_2$ | Fp.= 220° C |
| II-23 | 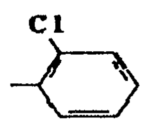 | $-CH(CH_3)_2$ | Fp.= 142° C |
| II-24 | 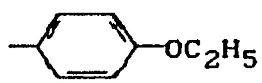 | $-CH(CH_3)_2$ | Fp.= 150° C |
| II-25 | 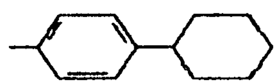 | $-CH(CH_3)_2$ | Fp.= 178° C |

| Beispiel Nr. | Ar | $R^1$ | Physikal. Konstanten |
|---|---|---|---|
| II-26 | 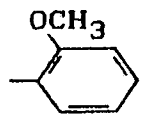 | $-CH(CH_3)_2$ | Fp.= 166° C |
| II-27 | 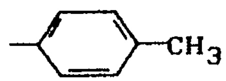 | $-CH(CH_3)_2$ | Fp.= 184° C |
| II-28 | 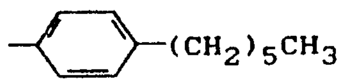 | $-C(CH_3)_3$ | Fp.= 114° C |
| II-29 | 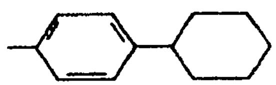 | $-C(CH_3)_3$ | Fp.= 186° C |
| II-30 | 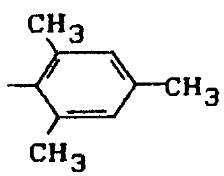 | $-CH_3$ | IR: 1722, 1660 $cm^{-1}$ |
| II-31 | 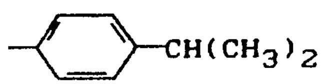 | $-C(CH_3)(C_2H_5)_2$ | Fp.= 172° C |

| Beispiel Nr. | Ar | $R^1$ | Physikal. Konstanten |
|---|---|---|---|
| II-32 | 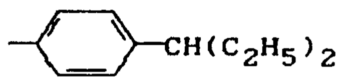 | $-C(CH_3)_3$ | Fp.= 136° C |
| II-33 | 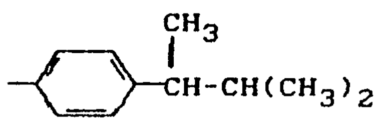 | $-C(CH_3)_3$ | Fp.= 184° C |
| II-34 | 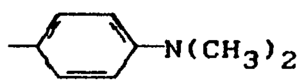 | $-C(CH_3)_3$ | Fp.= 238° C |
| II-35 | 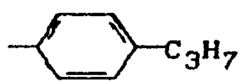 | $-C(CH_3)_3$ | Fp.= 220° C |
| II-36 | 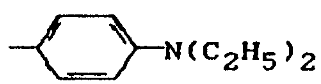 | $-C(CH_3)_3$ | Fp.= 240° C |
| II-37 | 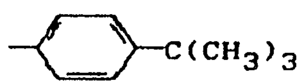 | $-C(CH_3)(C_2H_5)_2$ | Fp.= 206° C |

| Beispiel Nr. | Ar | $R^1$ | Physikal. Konstanten |
|---|---|---|---|
| II-38 | 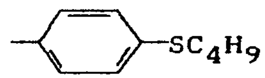 | $-C(CH_3)_3$ | IR: 1720, 1660 $cm^{-1}$ |
| II-39 | 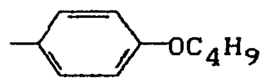 | $-C(CH_3)_3$ | IR: 1720, 1665, 1250 $cm^{-1}$ |
| II-40 | 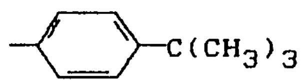 | $-C(CH_3)(C_2H_5)(C_4H_9)$ | IR: 1725, 1670, 760 $cm^{-1}$ |
| II-41 | 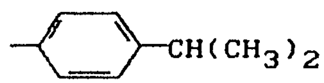 | $-C(CH_3)(C_2H_5)(C_4H_9)$ | IR: 1720, 1660, 1420, 1230 $cm^{-1}$ |
| II-42 | 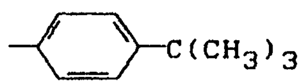 | $-C(CH_3)_2CH(CH_3)_2$ | IR: 1725, 1670, 1425 $cm^{-1}$ |
| II-43 | 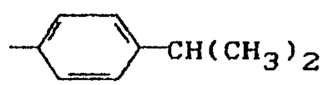 | $-C(CH_3)_2CH(CH_3)_2$ | IR: 1730, 1670, 1420 $cm^{-1}$ |

| Beispiel Nr. | Ar | $R^1$ | Physikal. Konstanten |
|---|---|---|---|
| II-44 | 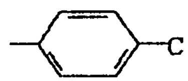$C(CH_3)_3$ | $-C(CH_3)_2CH_2F$ | Fp=178° C |
| II-45 | 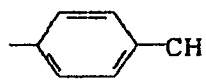$CH(CH_3)_2$ | $-C(CH_3)_2CH_2-OC_2H_5$ | Fp=124° C |
| II-46 | 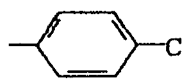$C_2H_5$ | $-C(CH_3)_3$ | Fp=162° C |
| II-47 | 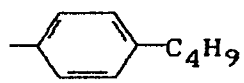$C_4H_9$ | $-C(CH_3)_3$ | Fp=168° C |
| II-48 | 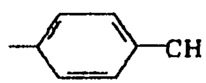$CH(CH_3)(C_2H_5)$ | $-C(CH_3)_3$ | Fp=156° C |
| II-49 | 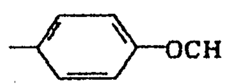$OCH(CH_2F)_2$ | $-C(CH_3)_3$ | Fp=164° C |

| Beispiel Nr. | Ar | $R^1$ | Physikal. Konstanten |
| --- | --- | --- | --- |
| II-50 | 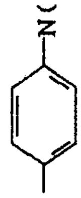$-N(C_4H_9)_2$ | $-C(CH_3)_3$ | IR: 1720, 1660, 750 $cm^{-1}$ |
| II-51 | 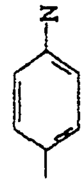$-N(C_3H_7)_2$ | $-C(CH_3)_3$ | IR: 1730, 1670 $cm^{-1}$ |
| II-52 | 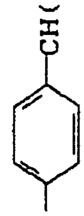$-CH(CH_3)_2$ | $-C_2H_5$ | Fp=200° C |

Herstellungsbeispiele der Verbindungen der Formel (IV)

(Beispiel Nr. IV-2)

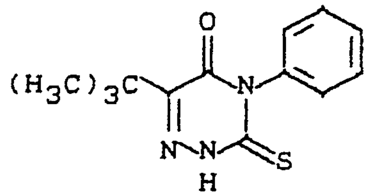

33,4 g (0,2 Mol) 4-Phenyl-thiosemicarbazid, 30,4 g (0,2 Mol) 2-Keto-3,3-dimethylbuttersäure-Na-Salz werden in einer Lösung von 55 ml konz, Salzsäure in 1 1 Wasser 8 Stunden unter Rückfluß gekocht. Nach dem Abkühlen wird das Reaktionsprodukt abgesaugt, mit viel Wasser gewaschen und getrocknet. Man erhält 39,5 g (76 % der Theorie) an gewünschtem Produkt. Physikalische Daten siehe Beispiel 2 in der folgenden Tabelle.

Analog dem angegebenen Beispiel werden die in der folgenden Tabelle genannten Verbindungen der Formel (IV) hergestellt:

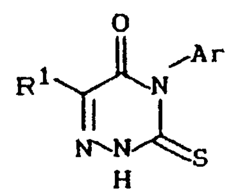 (IV)

| Beispiel Nr. | Ar | $R^1$ | Physikal. Konstanten |
|---|---|---|---|
| IV-1 | 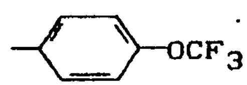 | $-C(CH_3)_3$ | Fp.= 186° C |
| IV-2 | 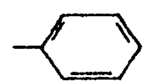 | $-C(CH_3)_3$ | Fp.= 222° C |
| IV-3 | 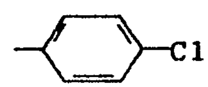 | $-C(CH_3)_3$ | Fp.= 186° C |
| IV-4 | 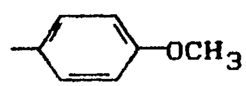 | $-C(CH_3)_3$ | Fp.= 214° C |

| Beispiel Nr. | Ar | $R^1$ | Physikal. Konstanten |
|---|---|---|---|
| IV-5 | 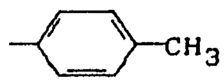 | $-C(CH_3)_3$ | Fp.= 196° C |
| IV-6 | 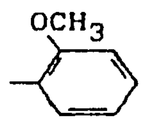 | $-C(CH_3)_3$ | Fp.= 220° C |
| IV-7 | 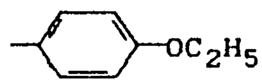 | $-C(CH_3)_3$ | Fp.= 196° C |
| IV-8 | 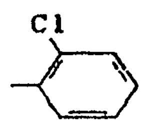 | $-C(CH_3)_3$ | Fp.= 200° C |
| IV-9 | 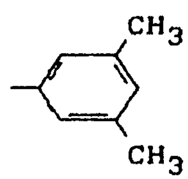 | $-C(CH_3)_3$ | Fp.= 184° C |

| Beispiel Nr. | Ar | $R^1$ | Physikal. Konstanten |
|---|---|---|---|
| IV-10 | 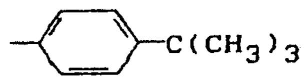 | $-C(CH_3)_3$ | Fp. = 228° C |
| IV-11 | 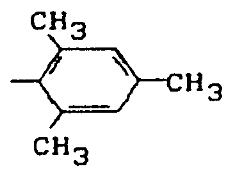 | $-C(CH_3)_3$ | Fp. = 260° C |
| IV-12 | 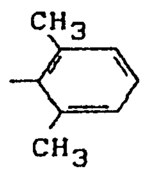 | $-C(CH_3)_3$ | Fp. = 192° C |
| IV-13 | 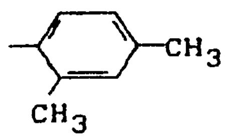 | $-C(CH_3)_3$ | Fp. = 208° C |
| IV-14 | 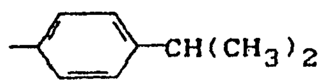 | $-C(CH_3)_3$ | Fp. = 202° C |

| Beispiel Nr. | Ar | $R^1$ | Physikal. Konstanten |
|---|---|---|---|
| IV-15 | 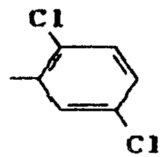 | $-C(CH_3)_3$ | Fp.= 216° C |
| IV-16 | 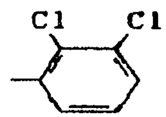 | $-C(CH_3)_3$ | Fp.= 212° C |
| IV-17 | 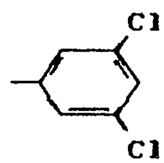 | $-C(CH_3)_3$ | Fp.= 188° C |
| IV-18 | 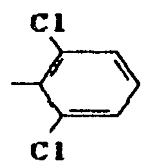 | $-C(CH_3)_3$ | Fp.= 186° C |
| IV-19 | 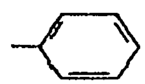 | $-CH(CH_3)_2$ | Fp.= 196° C |

| Beispiel Nr. | Ar | $R^1$ | Physikal. Konstanten |
|---|---|---|---|
| IV-20 | 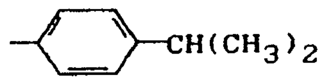 | $-CH(CH_3)_2$ | Fp.= 180° C |
| IV-21 | 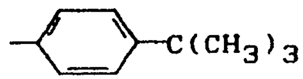 | $-CH(CH_3)_2$ | Fp.= 196° C |
| IV-22 | 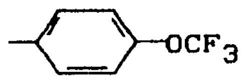 | $-CH(CH_3)_2$ | Fp.= 152° C |
| IV-23 | 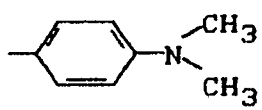 | $-CH(CH_3)_2$ | Fp.= 212° C |
| IV-24 | 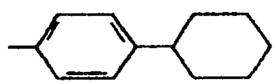 | $-CH(CH_3)_2$ | Fp.= 218° C |
| IV-25 | 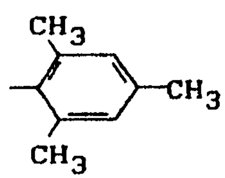 | $-CH_3$ | Fp.= 230° C (subl.) |
| IV-26 | 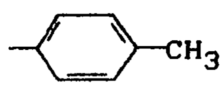 | $-CH(CH_3)_2$ | Fp.= 170° C |

| Beispiel Nr. | Ar | $R^1$ | Physikal. Konstanten |
| --- | --- | --- | --- |
| IV-27 | 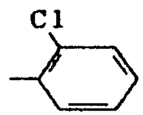 | $-CH(CH_3)_2$ | Fp.= 154° C |
| IV-28 | 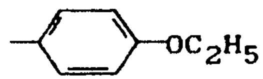 | $-CH(CH_3)_2$ | Fp.= 180° C |
| IV-29 | 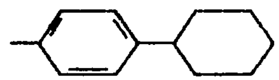 | $-C(CH_3)_3$ | Fp.= 189° C |
| IV-30 | 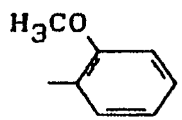 | $-CH(CH_3)_2$ | Fp.= 146° C |
| IV-31 | 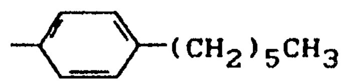 | $-C(CH_3)_3$ | Fp.= 182° C |
| IV-32 | 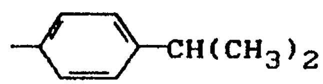 | $-C(CH_3)(C_2H_5)_2$ | Fp.= 154° C |

| Beispiel Nr. | Ar | $R^1$ | Physikal. Konstanten |
|---|---|---|---|
| IV-33 | 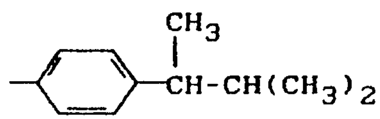 | $-C(CH_3)_3$ | Fp.= 162° C |
| IV-34 | 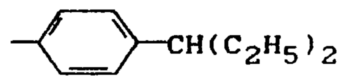 | $-C(CH_3)_3$ | Fp.= 182° C |
| IV-35 | 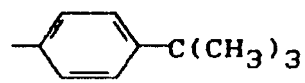 | $-C(CH_3)(C_2H_5)_2$ | Fp.= 166° C |
| IV-36 | 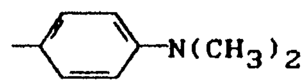 | $-C(CH_3)_3$ | Fp.= 210° C |
| IV-37 | 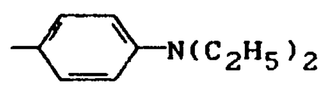 | $-C(CH_3)_3$ | Fp.= 250° C |
| IV-38 | 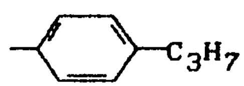 | $-C(CH_3)_3$ | Fp.= 233° C |

| Beispiel Nr. | Ar | $R^1$ | Physikal. Konstanten |
|---|---|---|---|
| IV-39 | 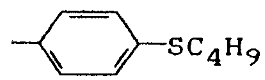 | $-C(CH_3)_3$ | IR: 1680-1710 $cm^{-1}$ |
| IV-40 | 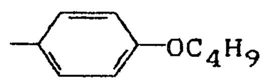 | $-C(CH_3)_3$ | IR: 1700 $cm^{-1}$ |
| IV-41 | 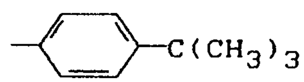 | $-C(CH_3)(C_2H_5)(C_4H_9)$ | IR: 1700, 1260 $cm^{-1}$ |
| IV-42 | 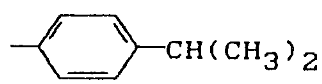 | $-C(CH_3)(C_2H_5)(C_4H_9)$ | IR: 1700, 1255 $cm^{-1}$ |
| IV-43 | 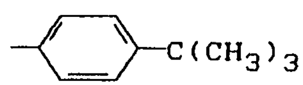 | $-C(CH_3)_2CH(CH_3)_2$ | IR: 1700, 1260 $cm^{-1}$ |
| IV-44 | 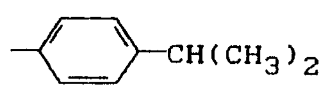 | $-C(CH_3)_2CH(CH_3)_2$ | IR: 1700,1260 $cm^{-1}$ |

| Beispiel Nr. | Ar | $R^1$ | Physikal. Konstanten |
|---|---|---|---|
| IV-45 | 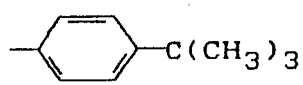 | $-C(CH_3)_2-CH_2F$ | Fp=224° C |
| IV-46 | 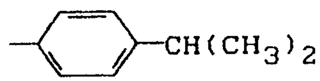 | $-C(CH_3)_2-CH_2OC_2H_5$ | Fp=122° C |
| IV-47 | 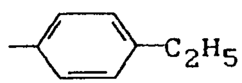 | $-C(CH_3)_3$ | IR: 1705, 1270 $cm^{-1}$ |
| IV-48 | 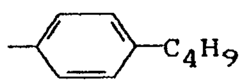 | $-C(CH_3)_3$ | IR: 1690-1720 $cm^{-1}$ |
| IV-49 | 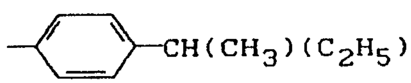 | $-C(CH_3)_3$ | Fp=180° C |
| IV-50 | 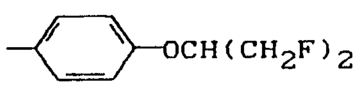 | $-C(CH_3)_3$ | IR: 1700, 1500, 1280, 1250 $cm^{-1}$ |

| Beispiel Nr. | Ar | $R^1$ | Physikal. Konstanten |
|---|---|---|---|
| IV-51 | 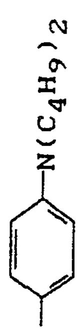 | $-C(CH_3)_3$ | Fp=179° C |
| IV-52 | 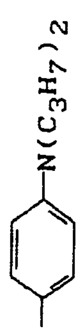 | $-C(CH_3)_3$ | Fp=177° C |
| IV-53 | 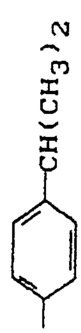 | $-C_2H_5$ | Fp=186° C |

## Anwendungsbeispiele

Als Vergleichssubstanz wurde die folgende Verbindung verwendet:

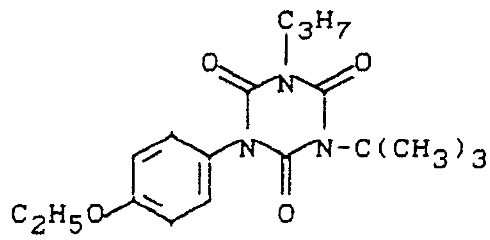

Beispiel A

Pyricularia-Test (Reis) / protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

## Patentansprüche

1. Trisubstituierte 1,2,4-Triazin-3,5-dione der allgemeinen Formel (I)

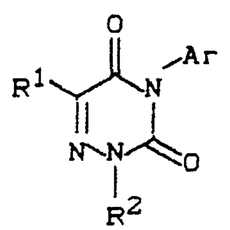

(I)

in welcher

Ar für gegebenenfalls substituiertes Aryl steht,

$R^1$ für einen gegebenenfalls substituierten aliphatischen oder cycloaliphatischen Rest steht und

$R^2$ für einen gegebenenfalls substituierten aliphatischen Rest steht.

2. Trisubstituierte 1,2,4-Triazin-3,5-dione gemäß Anspruch 1 der Formel (I), in welcher

Ar für Phenyl steht, welches gegebenenfalls ein-bis fünffach, gleich oder verschieden substituiert ist durch Halogen, Alkyl mit 1 bis 10 Kohlenstoffatomen, Alkenyl mit 2 bis 10 Kohlenstoffatomen, Alkinyl mit 2 bis 10 Kohlenstoffatomen, Halogenalkyl mit 1 bis 8 Kohlenstoffatomen und 1 bis 8 gleichen oder verschiedenen Halogenatomen, Alkoxy mit 1 bis 10 Kohlenstoffatomen, Halogenalkoxy mit 1 bis 8 Kohlenstoffatomen und 1 bis 8 gleichen oder verschiedenen Halogenatomen, Alkylthio mit 1 bis 10 Kohlenstoffatomen, Halogenalkylthio mit 1 bis 8 Kohlenstoffatomen und 1 bis 8 gleichen oder verschiedenen Halogenatomen, Amino, Monoalkylamino mit geradkettigen oder verzweigten Alkylresten mit 1 bis 6 Kohlenstoffatomen, Dialkylamino mit gleichen oder verschiedenen, geradkettigen oder verzweigten Alkylresten mit je 1 bis 6 Kohlenstoffatomen, Cycloalkyl oder Cycloalkenyl mit 3 bis 8 Kohlenstoffatomen, welche gegebenenfalls ein- bis sechsfach, gleich oder verschieden substituiert sind durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen oder Halogenalkoxy mit 1 bis 4 Kohlenstoffatomen und jeweils 1 bis 9 gleichen oder verschiedenen Halogenatomen, Alkylthio mit 1 bis 4

Kohlenstoffatomen und Halogenalkylthio mit 1 bis 4 Kohlenstoffatomen und 1 bis 5 gleichen oder verschiedenen Halogenatomen,

$R^1$ für geradkettiges oder verzweigtes Alkyl mit 1 bis 12 Kohlenstoffatomen, geradkettiges oder verzweigtes Alkenyl mit 3 bis 12 Kohlenstoffatomen oder geradkettiges oder verzweigtes Alkinyl mit 3 bis 12 Kohlenstoffatomen steht, welche gegebenenfalls ein- bis mehrfach, gleich oder verschieden substituiert sind durch Halogen, Alkoxy mit 1 bis 4 Kohlenstoffatomen oder Halogenalkoxy mit 1 bis 4 Kohlenstoffatomen und 1 bis 9 gleichen oder verschiedenen Halogenatomen, Alkylthio mit 1 bis 4 Kohlenstoffatomen, Halogenalkylthio mit 1 bis 4 Kohlenstoffatomen und 1 bis 9 gleichen oder verschiedenen Halogenatomen; weiterhin

$R^1$ für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 4 Kohlenstoffatomen, Halogenalkyl oder Halogenalkoxy mit 1 bis 4 Kohlenstoffatomen und 1 bis 9 gleichen oder verschiedenen Halogenatomen, Alkylthio mit 1 bis 4 Kohlenstoffatomen und Halogenalkylthio mit 1 bis 4 Kohlenstoffatomen und 1 bis 9 gleichen oder verschiedenen Halogenatomen,

$R^2$ für Alkyl mit 1 bis 10 Kohlenstoffatomen, Alkenyl mit 3 bis 10 Kohlenstoffatomen, Alkinyl mit 3 bis 10 Kohlenstoffatomen, Alkoxyalkyl mit je 1 bis 4 Kohlenstoffatomen im Alkoxy und im Alkylteil, Alkylthioalkyl mit je 1 bis 4 Kohlenstoffatomen im Alkylthioteil und im Alkylteil, Alkoxycarbonylalkyl mit 1 bis 4 Kohlenstoffatomen im Alkoxyteil und 1 bis 3 Kohlenstoffatomen im Alkylteil oder für Cyanoalkyl mit 1 bis 5 Kohlenstoffatomen im Alkylteil steht.

**3.** Trisubstituierte 1,2,4-Triazin-3,5-dione gemäß Anspruch 1 der Formel (I), in welcher

Ar für Phenyl steht, welches gegebenenfalls einbis vierfach, gleich oder verschieden substituiert sein kann durch Fluor, Chlor, Brom, Alkyl mit 1 bis 9 Kohlenstoffatomen, Alkenyl mit 2 bis 9 Kohlenstoffatomen, Alkinyl mit 2 bis 9 Kohlenstoffatomen, Halogenalkyl mit 1 bis 7 Kohlenstoffatomen und 1 bis 7 Chlor-und/oder Fluoratomen, Alkoxy mit 1 bis 9 Kohlenstoffatomen, Halogenalkoxy mit 1 bis 7 Kohlenstoffatomen und 1 bis 7 Chlor- und/oder Fluoratomen, Alkylthio mit 1 bis 9 Kohlenstoffatomen, Halogenalkylthio mit 1 bis 7 Kohlenstoffatomen und 1 bis 7 Chlor- und/oder Fluoratomen, Amino, Monoalkylamino mit geradkettigem oder verzweigtem Alkylrest mit 1 bis 5 Kohlenstoffatomen, Dialkylamino mit gleichen oder verschiedenen, geradkettigen oder verzweigten Alkylresten mit je 1 bis 5 Kohlenstoffatomen, Cycloalkyl oder Cycloalkenyl mit 3 bis 7 Kohlenstoffatomen, welches gegebenenfalls ein- bis fünffach, gleich oder verschieden substituiert ist durch Chlor und/oder Fluor, Alkyl mit 1 bis 3 Kohlenstoffatomen, Alkoxy mit 1 bis 3 Kohlenstoffatomen, Halogenalkoxy mit 1 bis 3 Kohlenstoffatomen und 1 bis 5 Chlor- und/oder Fluoratomen, Alkylthio mit 1 bis 3 Kohlenstoffatomen und Halogenalkylthio mit 1 bis 3 Kohlenstoffatomen und 1 bis 5 Chlor-und/oder Fluoratomen,

$R^1$ für geradkettiges oder verzweigtes Alkyl mit 1 bis 10 Kohlenstoffatomen, geradkettiges oder verzweigtes Alkenyl mit 3 bis 10 Kohlenstoffatomen oder geradkettiges oder verzweigtes Alkinyl mit 3 bis 10 Kohlenstoffatomen steht, welche jeweils gegebenenfalls ein- bis dreifach, gleich oder verschieden substituiert sind durch Chlor und/oder Fluor, Alkoxy mit 1 bis 3 Kohlenstoffatomen, Halogenalkoxy mit 1 bis 3 Kohlenstoffatomen und 1 bis 3 Chlor-und/oder Fluoratomen, Alkylthio mit 1 bis 3 Kohlenstoffatomen, Halogenalkylthio mit 1 bis 3 Kohlenstoffatomen und 1 bis 3 Chlor- und/ oder Fluoratomen;

$R^1$ weiterhin für Cycloalkyl mit 3 bis 7 Kohlen-stoffatomen steht, welches gegebenenfalls einbis sechsfach, gleich oder verschieden substituiert ist durch Fluor, Chlor, Alkyl mit 1 bis 3 Kohlenstoffatomen, Alkoxy mit 1 bis 3 Kohlenstoffatomen, Halogenalkoxy mit 1 bis 3 Kohlenstoffatomen und 1 bis 3 Chlor- und/oder Fluoratomen, Alkylthio mit 1 bis 3 Kohlenstoffatomen und Halogenalkylthio mit 1 bis 3 Kohlenstoffatomen und 1 bis 3 Chlor-und/oder Fluoratomen,

$R^2$ für Alkyl mit 1 bis 9 Kohlenstoffatomen, Alkenyl mit 3 bis 9 Kohlenstoffatomen, Alkinyl mit 3 bis 9 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht.

**4.** Trisubstituierte 1,2,4-Triazin-3,5-dione gemäß Anspruch 1 der Formel (I), in denen

Ar für Phenyl steht, welches gegebenenfalls einbis dreifach, gleich oder verschieden substituiert sein kann durch Fluor, Chlor, Brom, Alkyl mit 1 bis 8 Kohlenstoffatomen, Alkenyl mit 2 bis 8

Kohlenstoffatomen, Alkinyl mit 2 bis 8 Kohlenstoffatomen, Halogenalkyl mit 1 bis 6 Kohlenstoffatomen und 1 bis 5 Chlor-und/oder Fluoratomen, Alkoxy mit 1 bis 8 Kohlenstoffatomen, Halogenalkoxy mit 1 bis 6 Kohlenstoffatomen und 1 bis 5 Chlor-und/oder Fluoratomen, Alkylthio mit 1 bis 8 Kohlenstoffatomen, Halogenalkylthio mit 1 bis 6 Kohlenstoffatomen und 1 bis 5 Chlor-und/oder Fluoratomen, Amino, Monoalkylamino mit geradkettigem oder verzweigtem Alkylrest mit 1 bis 4 Kohlenstoffatomen, Dialkylamino mit gleichen oder verschiedenen, geradkettigen oder verzweigten Alkylresten mit je 1 bis 4 Kohlenstoffatomen, Cycloalkyl oder Cycloalkenyl mit 3 bis 6 Kohlenstoffatomen, welche gegebenenfalls ein- bis vierfach, gleich oder verschieden substituiert sind durch Chlor, Fluor, Alkyl mit 1 bis 3 Kohlenstoffatomen, Alkoxy mit 1 bis 3 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen und Halogenalkylthio mit 1 oder 2 Kohlenstoffatomen und 1 bis 5 Chlor -und/oder Fluoratomen,

$R^1$ für geradkettiges oder verzweigtes Alkyl mit 1 bis 9 Kohlenstoffatomen, geradkettiges oder verzweigtes Alkenyl mit 3 bis 9 Kohlenstoffatomen oder geradkettiges oder verzweigtes Alkinyl mit 3 bis 9 Kohlenstoffatomen steht, welche jeweils gegebenenfalls ein- bis zweifach, gleich oder verschieden substituiert sind durch Chlor, Fluor, Alkoxy mit 1 bis 3 Kohlenstoffatomen, Halogenalkoxy mit 1 bis 3 Kohlenstoffatomen und 1 bis 3 Chlor- und/oder Fluoratomen, Alkylthio mit 1 bis 3 Kohlenstoffatomen, Halogenalkylthio mit 1 bis 3 Kohlenstoffatomen und 1 bis 3 Chlor-und/oder Fluoratomen;

$R^1$ weiterhin für Cycloalkyl mit 3 bis 7 Kohlenstoffatomen steht, welches gegebenenfalls ein-bis fünffach, gleich oder verschieden substituiert ist durch Fluor, Chlor, Alkyl mit 1 oder 2 Kohlenstoffatomen, Alkoxy mit 1 oder 2 Kohlenstoffatomen, Halogenalkoxy mit 1 oder 2 Kohlenstoffatomen und 1 oder 4 Chlor- und/ oder Fluoratomen, Alkylthio mit 1 oder 2 Kohlenstoffatomen und Halogenalkylthio mit 1 bis 2 Kohlenstoffatomen und 1 bis 3 Chlor- und/oder Fluoratomen,

R2 für Alkyl mit 1 bis 8 Kohlenstoffatomen, Alkenyl mit 3 bis 8 Kohlenstoffatomen, Alkinyl mit 3 bis 8 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanalkyl mit 1 bis 3 Kohlenstoffatomen im Alkylteil steht.

**5.** Verfahren zur Herstellung von trisubstituierten 1,2,4-Triazin-3,5-dionen der allgemeinen Formel (I)

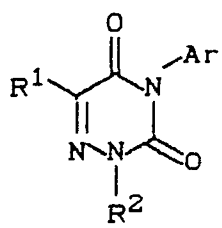

(I)

in welcher

Ar für gegebenenfalls substituiertes Aryl steht,

$R^1$ für einen gegebenenfalls substituierten aliphatischen oder cycloaliphatischen Rest steht und

$R^2$ für einen gegebenenfalls substituierten aliphatischen Rest steht,

dadurch gekennzeichnet, daß man disubstituierte 1,2,4-Triazin-3,5-dione der allgemeinen Formel (II)

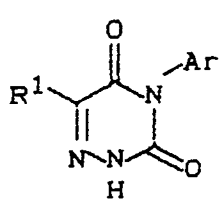

(II)

wobei Ar und $R^1$ die oben angegebene Bedeutung haben, mit Verbindungen der allgemeinen Formel

(III)

$R^2$ - X (III)

in welcher

$R^2$ die oben angegebene Bedeutung hat und

X für eine Abgangsgruppe steht,

gegebenenfalls in Gegenwart eines Verdünnungsmittels und gegebenenfalls in Gegenwart eines Säurebindemittels umsetzt.

**6.** Schädlingsbekämpfungsmittel, gekennzeichnet durch einen Gehalt an mindestens einem trisubstituierten 1,2,4-Triazin-3,5-dion der Formel (I) nach den Ansprüchen 1 und 5.

**7.** Verwendung von mindestens einem trisubstituierten 1,2,4-Triazin-3,5-dion der Formel (I) nach den Ansprüchen 1 und 5 zur Bekämpfung von Schädlingen.

**8.** Verfahren zur Bekämpfung von Schädlingen, dadurch gekennzeichnet, daß man mindestens ein trisubstituiertes 1,2,4-Triazin-3,5-dion der Formel (I) nach Ansprüchen 1 und 5 auf Schädlinge und/oder ihren Lebensraum einwirken läßt.

**9.** Verfahren zur Herstellung von Schädlingsbekämpfungsmitteln, dadurch gekennzeichnet, daß man mindestens ein trisubstituiertes 1,2,4-Triazin-3,5-dion der Formel (I) nach den Ansprüchen 1 und 5 mit Streckmitteln und/oder oberflächenaktiven Mitteln vermischt.

**10.** Disubstituierte 1,2,4-Triazin-3,5-dione der Formel (IIa)

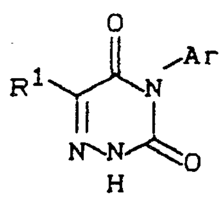

(IIa)

in welcher

Ar für gegebenenfalls substituiertes Aryl steht,

$R^1$ für einen gegebenenfalls substituierten aliphatischen oder cycloaliphatischen Rest steht,

ausgenommen die Verbindungen, in denen Ar für Phenyl oder 4-Methylphenyl und $R^1$ gleichzeitig für Methyl stehen.

**11.** Disubstituierte 3-Thioxo-1,2,4-triazin-5-one der Formel (IVa)

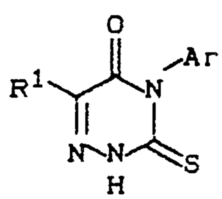

(IVa)

in welcher

Ar für gegebenenfalls substituiertes Aryl steht und

$R^1$ für einen gegebenenfalls substituierten aliphatischen oder cycloaliphatischen Rest steht, ausgenommen die Verbindungen, in denen Ar für Phenyl, 4-Methylphenyl, 3-Methylphenyl, 2-Methoxyphenyl, 3-Methoxyphenyl, 2-Ethoxyphenyl oder 4-Chlorphenyl und gleichzeitig $R^1$ für Methyl stehen.

**12.** Verwendung der Verbindungen der Formeln (IIa) und (IVa) gemäß den Ansprüchen 10 und 11 als

Ausgangsverbindungen für hochaktive Wirkstoffe,